# EUROPEAN PATENT SPECIFICATION

(11) **EP 3 408 282 B1**
(45) Date of publication and mention of the grant of the patent: **01.12.2021**
(21) Application number: 17704682.8
(22) Date of filing: 27.01.2017
(51) Int. Cl.: C07K 14/415, C12N 15/82, C07K 14/405, C12N 9/02

(54) **INCREASED TRIACYLGLYCEROL PRODUCTION IN MICROALGAE**
ERHÖHTE TRIACYLGLYCEROLHERSTELLUNG IN MIKROALGEN
PRODUCTION DE TRIACYLGLYCÉROL ACCRUE DANS DES MICRO-ALGUES

(30) Priority: 29.01.2016 WO PCT/EP2016/153390
(43) Date of publication of application: 05.12.2018
(73) Proprietor: Total Raffinage Chimie, 92400 Courbevoie (FR)
(72) Inventor: MARECHAL, Eric, 38000 Grenoble (FR); DOLCH, Lina, 40591 Düsseldorf (DE)
(74) Representative: Blaise, Lucie
(86) International application number: PCT/EP2017/051823
(87) International publication number: WO 2017/129777

(56) References cited:
- WO-A1-2013/126076
- WO-A2-2009/125401
- WO-A2-2015/008160
- VARDI ASSAF ET AL: "A Diatom Gene Regulating Nitric-Oxide Signaling and Susceptibility to Diatom-Derived Aldehydes", CURRENT BIOLOGY, vol. 18, no. 12, June 2008 (2008-06), pages 895-899, XP028813755, ISSN: 0960-9822, DOI: 10.1016/J.CUB.2008.05.037
- VALERIA DI DATO ET AL: "Transcriptome sequencing of three Pseudo-nitzschia species reveals comparable gene sets and the presence of Nitric Oxide Synthase genes in diatoms", SCIENTIFIC REPORTS, vol. 5, 20 July 2015 (2015-07-20), page 12329, XP055264077, DOI: 10.1038/srep12329
- N. FORESI ET AL: "Characterization of a Nitric Oxide Synthase from the Plant Kingdom: NO Generation from the Green Alga Ostreococcus tauri Is Light Irradiance and Growth Phase Dependent", THE PLANT CELL, vol. 22, no. 11, 1 November 2010 (2010-11-01), pages 3816-3830, XP055264116, US ISSN: 1040-4651, DOI: 10.1105/tpc.109.073510
- GOMMA AHMED E ET AL: "Improvement in Oil Production by Increasing Malonyl-CoA and Glycerol-3-Phosphate Pools inScenedesmus quadricauda", INDIAN JOURNAL OF MICROBIOLOGY, HISAR, IN, vol. 55, no. 4, 7 August 2015 (2015-08-07) , pages 447-455, XP035580072, ISSN: 0046-8991, DOI: 10.1007/S12088-015-0546-4 [retrieved on 2015-08-07]

## Description

### TECHNICAL FIELD

The application generally relates to bioproduction of molecules of interest in micro-organisms particularly in microalgae. In particular, the application relates to methods for increasing triacylglycerol production in microalgae.

### BACKGROUND

Microalgae have the ability to accumulate significant amounts of lipids, primarily in the form of triacylglycerol (TAG), especially under stress conditions like nutrient limitation, temperature, pH, or light stress. Nitrogen deprivation is considered a critical factor affecting lipid metabolism in microalgae. Nitrogen deprivation limits amino acid production and decreases protein synthesis, thereby impairing growth and photosynthesis, which leads to an accumulation of lipids, in particular TAG, which are used as carbon and energy provisions.

The ability of microalgae such as *Phaeodactylum tricornutum* to accumulate TAG has triggered their exploitation as host for fatty acid production, e.g. for biofuel production, for chemical applications or in food industry. P. *tricornutum* for instance is currently used for the industrial production of omega-3 polyunsaturated fatty acids.

Approaches to enhance TAG accumulation can rely on nutrient starvation such as nitrogen starvation, in particular the reduction of nitrate (NO₃⁻) availability in the medium. Disrupting the assimilation pathway of NO₃⁻ by genetic engineering has therefore been considered as a way to trigger TAG accumulation, and reducing the expression of a nitrate reductase from *P. tricornutum* has been shown to promote TAG accumulation per cell (Levitan et al. 2015 Proc Natl Acad Sci U S A 112:412-417, US 2012/0282676). Other attempts to promote TAG accumulation include the stimulation of fatty acid and TAG biosynthesis, the blocking of pathways that divert carbon to alternative metabolic routes and eventually the arrest of TAG catabolism through genetic engineering of the microalgae (Maréchal 2015 In Techniques de I' Ingénieur. In 186: 1-19 and US 2014/0256927). Various strategies can also be combined.

The implementation of microalgae in industrial processes is currently based on a two-step process: first, the biomass grows using the nutrients provided in the culture medium and second, growth is slowed down or stopped by nutrient starvation, e.g. nitrogen starvation.

In the above second step, TAG accumulation occurs, whereas there is virtually no TAG accumulation during the first cell growth step.

There is a need for alternative methods for enhancing triacylglycerol accumulation in microalgae, preferably without compromising cell growth and biomass yield so as to improve overall lipid productivity. In other words, it would be advantageous to implement a method in which TAG can accumulate during a cell growth step.

### SUMMARY OF THE INVENTION

The present invention is based, at least in part, on the discovery that exposure of microalgae to nitric oxide (NO) increases the production of certain molecules of interest; in particular, it was discovered that exposure of microalgae to nitric oxide (NO) triggers TAG accumulation. More particularly, the present inventors have found that microalgae that are genetically engineered to induce or increase NO production, in particular by (over)expression of a gene encoding a NOA protein accumulate significantly more TAG compared to microalgae wherein NO production has not been modulated. In addition, it was found that cell concentration was not substantially impacted by modulation of NO production in the genetically engineered strains, such that production of TAG can occur during the growth phase of the microalgae, which can improve overall productivity of TAG.

The present invention is in particular captured by any one or any combination of one or more of the below numbered aspects and embodiments (i) to (xvi) wherein:
(i) A method for increasing the production of triacylglycerol (TAG) in a microalga, said method comprising:
   culturing a recombinant microalga which has been transformed with a recombinant nucleic acid encoding a NOA protein under conditions suitable for the production or overproduction of NO by said microalga, so as to enhance the production of said triacylglycerol,
   wherein the triacylglycerol content is increased in said recombinant microalga to at least 150%, preferably at least 200%, compared to a corresponding microalga wherein the NO production pathway was not modulated.
(ii) The method according to (i), wherein said NOA protein is the *Phaeodactylum tricornutum* NOA (*Pt*NOA) protein having an amino acid sequence of SEQ ID NO:2 or a functional variant thereof that has an amino acid sequence of at least 80% sequence identity with SEQ ID NO:2.
(iii) The method according to (i), wherein said NOA protein is the *Nannochloropsis gaditana* NOA (*Ng*NOA) protein having the amino acid sequence of SEQ ID NO:4 or a functional variant thereof that has an amino acid sequence of at least 80% sequence identity with SEQ ID NO:4.
(iv) The method according to any one of (i) to (iii), wherein the microalga is selected from the Chromalveolata.
(v) The method according to (iv), wherein the microalga is selected from the Bacillariophyceae or the Eustigmatophyceae.
(vi) The method according to (v), wherein the microalga is *Phaeodactylum tricornutum.*
(vii) The method according to any one of (i) to (vi), wherein the production of triacylglycerol occurs during the growth of the recombinant microalga.
(viii) Use of a recombinant microalga which has been transformed with a recombinant nucleic acid encoding a NOA protein for the production of TAGs, wherein the triacylglycerol content is increased in said recombinant microalga to at least 150%, preferably at least 200%, compared to a corresponding microalga wherein the NO production pathway was not modulated.
(ix) Use according to (viii) for the production of fatty acids, hydrocarbons or fatty alcohols.
(x) Use according to any one of (viii) or (ix) for biofuel production or for the production of cosmetics.
(xi) A recombinant microalga, which has been transformed with a recombinant nucleic acid encoding a *Pt*NOA homologthat is from *Nannochloropsis gaditana* and that has the amino acid sequence of SEQ ID NO:4, wherein the triacylglycerol content is increased in said recombinant microalga to at least 150%, preferably at least 200%, compared to a corresponding microalga wherein the NO production pathway was not modulated.

### BRIEF DESCRIPTION OF THE FIGURES

The teaching of the application is illustrated by the following Figures which are to be considered as illustrative only and do not in any way limit the scope of the claims.
**Figure 1:** (A) Sequence alignment of *Pt*NOA (SEQ ID NO:2) and *Ng*NOA (SEQ ID NO:4). (B) Nucleotide sequence coding for *Ng*NOA (SEQ ID NO:3).
**Figure 2:** Vector map (A) and vector sequence (B, SEQ ID NO:6) of the pH4-GUS vector containing the NOA gene of P. *tricornutum* (pH4-NOAOE). (A) The *Pt*NOA gene was cloned under the control of the constitutive histone 4 promoter (H4prom). The vector further contained a gene coding for resistance to zeocin (*shble*), allowing selection of transformed cells. **(B)** The ATG and TAG of the *Pt*NOA gene are underlined. The coding sequence of *Pt*NOA is underlined (SEQ ID NO:1). TheXbal and *EcoR*I sites used for cloning are in bold. The intron is in italics.
**Figure 3: Exemplary NOA overexpressing *P. tricornutum* strains.** Two independent *Pt*NOA overexpressing strains of P. *tricornutum* are shown: NOAE4 and NOAEf. pH4 is a control P. *tricornutum* strain that was transformed with an empty vector. NOA expression level was measured in three biological replicates, and normalized using the housekeeping genes 30S Ribosomal Protein Subunit (RPS) and tubulin (TUB).
**Figure 4: Increased level of NO in *P. tricornutum* strains overexpressing NOA.** Levels of NO were measured in 500 µl cultures using the NO-indicator DAF-FM and are expressed in relative fluorescence unit. Measurements were performed in triplicate at the indicated time points **(A)** or after three hours of incubation with DAF-FM **(B).**
**Figure 5:** Increased TAG per cell **(A)** and TAG productivity **(B)** in cultures of P. *tricornutum* performed in 50 mL culture flasks. Nile Red fluorescence was measured after 3 days of culture and was increased in the NOA overexpression strains (NOAOE4, NOAOEf) compared to a strain transformed with an empty vector (PH4).
**Figure 6:** Increased TAG productivity in cultures of P. *tricornutum* overexpressing *Pt*NOA (Pt1 NOA OE4) or WT (Pt1 WT) grown in a photobioreactor in presence or absence of CO₂ supplies (air, 1.5% CO₂ and 0.5% CO₂) and in different media. TAG was quantified by mass spectrometry analysis. The relative productivity was defined by the level of TAG in collected cells, after extraction of lipids, purification of TAG and analysis by mass spectrometry.
**Figure 7:** Cell concentration in cultures of P. *tricornutum* performed in 10 mL culture flasks for *Pt*NOA overexpressing strains (NOAOE4, NOAOEf) compared to a strain transformed with an empty vector (PH4).
**Figure 8:** SEQ ID NO:7

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. Where reference is made to embodiments as comprising certain elements or steps, this encompasses also embodiments which consist essentially of the recited elements or steps. The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" refers is itself also specifically, and preferably, disclosed.

As used herein, the terms "microbial", "microbial organism" or "micro-organism" are intended to mean any organism that exists as a microscopic cell that is included within the domains of archaea, bacteria or eukaryotes. Therefore, the term is intended to encompass prokaryotic or eukaryotic cells or organisms having a microscopic size and includes bacteria, archaea and eubacteria such as cyanobacteria of all species as well as eukaryotic micro-organisms such as fungi, including yeasts, and algae. The term also includes cell cultures of any species.

The term "microalga" or "microalgae" (plural) as used herein refers to microscopic alga(e). "Microalgae" encompass, without limitation, organisms within (i) several eukaryotic phyla, including the Rhodophyta (red algae), Chlorophyta (green algae), Dinoflagellata, Haptophyta, (ii) several classes from the eukaryotic phylum Heterokontophyta which includes, without limitation, the classes Bacillariophycea (diatoms), Eustigmatophycea, Phaeophyceae (brown algae), Xanthophyceae (yellow-green algae) and Chrysophyceae (golden algae), and (iii) the prokaryotic phylum Cyanobacteria (blue-green algae). The term "microalgae" includes for example genera selected from: *Achnanthes, Amphora, Anabaena, Anikstrodesmis, Arachnoidiscusm, Aster, Botryococcus, Chaetoceros, Chlamydomonas, Chlorella, Chlorococcum, Chorethron, Cocconeis, Coscinodiscus, Crypthecodinium, Cyclotella, Cylindrotheca, Desmodesmus, Dunaliella, Emiliana, Euglena, Fistulifera, Fragilariopsis, Gyrosigma, Hematococcus, Isochrysis, Lampriscus, Monochrysis, Monoraphidium, Nannochloris, Nannochloropsis, Navicula, Neochloris, Nephrochloris, Nephroselmis, Nitzschia, Nodularia, Nostoc, Odontella, Oochromonas, Oocystis, Oscillartoria, Pavlova, Phaeodactylum, Playtmonas, Pleurochrysis, Porhyra, Pseudoanabaena, Pyramimonas, Scenedesmus, Schyzochitrium, Stichococcus, Synechococcus, Synechocystis, Tetraselmis, Thalassiosira,* and *Trichodesmium.*

The term "transformation" means introducing an exogenous nucleic acid into an organism so that the nucleic acid is replicable, either as an extrachromosomal element or by chromosomal integration.

The terms "genetically engineered" or "genetically modified" or "recombinant" as used herein with reference to a host cell, in particular a micro-organism such as a microalga, denote a non-naturally occurring host cell, as well as its recombinant progeny, that has at least one genetic alteration not found in a naturally occurring strain of the referenced species, including wild-type strains of the referenced species. Such genetic modification is typically achieved by technical means (i.e. non-naturally) through human intervention and may include, e.g., the introduction of an exogenous nucleic acid and/or the modification, over-expression, or deletion of an endogenous nucleic acid.

The term "exogenous" or "foreign" as used herein is intended to mean that the referenced molecule, in particular nucleic acid, is not naturally present in the host cell.

The term "endogenous" or "native" as used herein denotes that the referenced molecule, in particular nucleic acid, is present in the host cell.

By "recombinant nucleic acid" when referring to a nucleic acid in a recombinant host cell, in particular a recombinant micro-organism such as a recombinant microalga, is meant that at least part of said nucleic acid is not naturally present in the host cell in the same genomic location. For instance a recombinant nucleic acid can comprise a coding sequence naturally occurring in the host cell under control of an exogenous promotor, or it can be an additional copy of a gene naturally occurring in the host cell, or a recombinant nucleic acid can comprise an exogenous coding sequence under the control of an endogenous promoter.

By "nucleic acid" is meant oligomers and polymers of any length composed essentially of nucleotides, e.g., deoxyribonucleotides and/or ribonucleotides. Nucleic acids can comprise purine and/or pyrimidine bases and/or other natural (e.g., xanthine, inosine, hypoxanthine), chemically or biochemically modified (e.g., methylated), non-natural, or derivatised nucleotide bases. The backbone of nucleic acids can comprise sugars and phosphate groups, as can typically be found in RNA or DNA, and/or one or more modified or substituted sugars and/or one or more modified or substituted phosphate groups. Modifications of phosphate groups or sugars may be introduced to improve stability, resistance to enzymatic degradation, or some other useful property. A "nucleic acid" can be for example doublestranded, partly double stranded, or single-stranded. Where single-stranded, the nucleic acid can be the sense strand or the antisense strand. The "nucleic acid" can be circular or linear. The term "nucleic acid" as used herein preferably encompasses DNA and RNA, specifically including genomic, hnRNA, pre-mRNA, mRNA, cDNA, recombinant or synthetic nucleic acids, including vectors.

By "encoding" is meant that a nucleic acid sequence or part(s) thereof corresponds, by virtue of the genetic code of an organism in question, to a particular amino acid sequence, e.g., the amino acid sequence of a desired polypeptide or protein. By means of example, nucleic acids "encoding" a particular polypeptide or protein, e.g. an enzyme, may encompass genomic, hnRNA, pre-mRNA, mRNA, cDNA, recombinant or synthetic nucleic acids.

Preferably, a nucleic acid encoding a particular polypeptide or protein may comprise an open reading frame (ORF) encoding said polypeptide or protein. An "open reading frame" or "ORF" refers to a succession of coding nucleotide triplets (codons) starting with a translation initiation codon and closing with a translation termination codon known *per se,* and not containing any internal in-frame translation termination codon, and potentially capable of encoding a polypeptide or protein. Hence, the term may be synonymous with "coding sequence" as used in the art.

The terms "polypeptide" and "protein" are used interchangeably herein and generally refer to a polymer of amino acid residues linked by peptide bonds, and are not limited to a minimum length of the product. Thus, peptides, oligopeptides, polypeptides, dimers (hetero- and homo-), multimers (hetero- and homo-), and the like, are included within the definition. Both full-length proteins and fragments thereof are encompassed by the definition. The terms also include post-expression modifications of the polypeptide, for example, glycosylation, acetylation, phosphorylation, etc. Furthermore, as used herein, the terms also refer to such when including modifications, such as deletions, additions and substitutions (e.g., conservative in nature), to the sequence of a native protein or polypeptide.

The term "variant", when used in connection to a protein, such as an enzyme, for example as in "a variant of protein X", refers to a protein, such as an enzyme, that is altered in its sequence compared to protein X, but that retains the activity of protein X, such as the enzymatic activity (i.e. a functional variant or homolog). More particularly a functional variant of protein X involved in the production of NO, such as a NOA protein, is capable of ensuring at least 60% of the activity of protein X in the production of NO. More particularly, the effect of a homolog or functional variant on NO production activity can be determined by measuring NO levels in a micro-organism. For example, NO levels can be measured using the fluorophore 4-amino-5-methylamino-2',7'-difluororescein diacetate (DAF-FM), which allows detection of nitric oxide (ONOO⁻) which is in equilibrium with NO (St Laurent et al. 2015 Methods Mol Biol. 1220:339-345). The functional variant can be a non-natural variant. Alternatively, the functional variant can be a homolog. Preferably, such variant would show at least 80%, more preferably at least 85%, even more preferably at least 90%, and yet more preferably at least 95% such as at least 96%, at least 97%, at least 98% or at least 99% sequence identity to the reference protein, preferably calculated over the entire length of the sequence. The sequence changes may be naturally occurring, for example, due to the degeneracy of the genetic code, or may be introduced artificially, for example by targeted mutagenesis of the respective sequence. Such techniques are well known to the skilled person.

The term "homolog" as used herein in connection to a protein, such as an enzyme, for example as in "a homolog of protein X" refers to the fact that the protein differs from protein X in its sequence, but that retains the activity or protein X, such as the enzymatic activity as detailed above, and originates from another species, i.e. is a naturally occurring sequence. A homolog of protein X can be identified by the skilled person by pairwise search methods such as BLAST and checking of the corresponding activity.

As used herein, the terms "identity" and "identical" and the like are used interchangeably with the terms "homology" and "homologues" and the like herein and refer to the sequence similarity between two polymeric molecules, e.g., between two nucleic acid molecules or polypeptides. Methods for comparing sequences and determining sequence identity are well known in the art. By means of example, percentage of sequence identity refers to a percentage of identical nucleic acids or amino acids between two sequences after alignment of these sequences. Alignments and percentages of identity can be performed and calculated with various different programs and algorithms known in the art. Preferred alignment algorithms include BLAST (Altschul, 1990; available for instance at the NCBI website) and Clustal (reviewed in Chenna, 2003; available for instance at the EBI website). Preferably, BLAST is used to calculate the percentage of identity between two sequences, such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62, cost to open a gap = 11, cost to extend a gap = 1, expectation value = 10.0, word size = 3).

As used herein, the term "molecules of interest" refers to any molecule which can be produced by micro-organisms, including but not limited to molecules derived from the acetyl-CoA pool in a micro-organism. Such molecules of interest include, without limitation, hydrocarbons, fatty acids and lipids. Such molecules of interest can be recovered from the micro-organism or its culture medium, and then used in certain applications.

As used herein, the term "lipid metabolic pathway" refers to any pathway in a micro-organism comprised between acetyl-CoA and lipids (it being understood that acetyl-CoA is included in such lipid metabolic pathway). Said term hence encompasses without limitation fatty acid synthesis pathways, pathways ensuring the assembly of triacylglycerols (TAGs) or the conversion of any lipids to TAGs, and pathways degrading TAGs (beta-oxidation).

As used herein, "triacylglycerols", also referred to as "triacylglycerides" or "TAG" are esters resulting from the esterification of the three hydroxyl groups of glycerol, with three fatty acids.

The present application generally relates to production of molecules of interest, in particular production of molecules of the lipid metabolic pathway, including production of triacylglycerol (TAG) and any intermediates in the lipid metabolic pathway, in micro-organisms, in particular in microalgae. The application is further directed to the production of biomolecules derived from said molecules of the lipid metabolic pathway.

More particularly, the application provides methods for increasing TAG production in micro-organisms, in particular microalgae, by genetically engineering the micro-organisms, in particular the microalgae, to produce or overproduce nitric oxide (NO). The application also encompasses the recombinant micro-organisms as well as their use, e.g. for fatty acid production.

It has been surprisingly found that exposure of microalgae such as *Phaeodactylum tricornutum* to nitric oxide (NO) triggers TAG accumulation in the microalgae. The present inventors have found that genetic engineering of microalgae to produce or overproduce NO by transformation with a recombinant nucleic acid encoding a NOA protein (such as the NOA protein of *Phaeodactylum tricornutum*), results in increased TAG production in said recombinant microalgae.

Accordingly, in an aspect, the application provides a method for increasing the production of molecules of interest in a micro-organism, in particular a microalga, said method comprising culturing a recombinant micro-organism, in particular a recombinant microalga, which has been genetically engineered to produce or overproduce NO under conditions suitable to produce or overproduce NO in said recombinant micro-organism. Thus, disclosed herein is a method for the production of molecules of interest, which encompasses the steps of (i) genetically engineering a micro-organism, in particular a microalga, to produce or overproduce NO; and (ii) culturing the recombinant micro-organism, in particular the recombinant microalga, obtained in step (b) so as to allow the production of said molecules of interest.

The molecules of interest may be molecules of the lipid metabolic pathway or biomolecules derived from said molecules and the production of such molecules of interest is increased according to the methods disclosed herein. For example, the molecules of interest may be lipids, in particular triacylglycerols (TAGs).

The recombinant micro-organism may have been engineered to express or overexpress a protein involved in an NO production pathway.

Preferably, the recombinant micro-organism has been transformed with a recombinant nucleic acid encoding a protein involved in an NO production pathway.

Accordingly, the method disclosed herein may encompass transforming the micro-organism with a recombinant nucleic acid encoding a protein involved in an NO production pathway, and culturing the recombinant micro-organism so obtained under conditions suitable to produce or overproduce NO in said recombinant micro-organism so as to allow production of the desired molecule or biomolecule by the micro-organism.

NO production differs from organism to organism. Some diatoms such as *Pseudo-nitzschia multistriata* contain a nitric oxide synthase (NOS) (Di Dato et al. 2015 Scientific Reports 5:12329). In the pennate diatom *Phaeodactylum tricornutum,* NO production depends on the activity of a protein called NOA (Vardi et al. 2008). Any protein or enzyme involved in NO production is envisaged herein for (over)expression in the recombinant micro-organisms described herein.

In the method of the invention, the protein involved in the NO production pathway is a NOA protein. In particular embodiments, the NOA protein is an NOA protein of microbial origin, such as from a microalga or diatom. In particular embodiments, the protein involved in NO production is the NOA protein of a *Phaeodactylum* more particularly of P. *tricornutum,* also referred to herein as PtNOA, or a variant or a homolog thereof. In particular embodiments, of the present invention, the homologue of PtNOA originates from a microalga. As used herein the term "PtNOA" refers to a protein having an amino acid sequence of SEQ ID NO:2. Preferably, the variants have a sequence substantially identical to SEQ ID NO:2, or a sequence having at least about 70%, preferably at least about 80%, more preferably at least about 85%, 90% or 95%, even more preferably at least about 96%, 97%, 98% or 99% sequence identity to SEQ ID NO:2.

Accordingly, in embodiments, the recombinant microalga has been transformed with a recombinant nucleic acid encoding a NOA protein. In particular embodiments, the recombinant nucleic acid encoding a NOA protein encodes a NOA protein from a *Phaeodactylum* species or a variant or a homolog thereof. In further embodiments, the recombinant microalga comprises a recombinant nucleic acid encoding a *Phaeodactylum tricornutum* NOA protein or a homolog or functional variant thereof. In particular embodiments, of the present invention, the homologue of PtNOA originates from a microalga. Most particularly, the recombinant microalga is transformed with a recombinant nucleic acid comprising the sequence of SEQ ID NO:1 or a sequence substantially identical to SEQ ID NO:1, or a sequence having at least about 70%, preferably at least about 80%, more preferably at least about 85%, 90% or 95%, even more preferably at least about 96%, 97%, 98% or 99% sequence identity to SEQ ID NO:1.

The application also envisages the use of recombinant nucleic acids encoding homologs of the identified *Phaeodactylum tricornutum* NOA protein. For instance the present inventors have identified a genomic sequence in *Nannochloropsis gaditana* (SEQ ID NO:3) encoding a protein of SEQ ID NO:4 that is homologous to *Pt*NOA (Fig. 1A), which protein is referred to herein as *Ng*NOA. In particular embodiments, the recombinant nucleic acid encoding a NOA protein encodes a NOA protein from *Nannochloropsis gaditana* or a variant thereof, i.e. having an amino acid sequence of SEQ ID NO:4 (i.e. *Ng*NOA) or a variant thereof. Indeed, also envisaged herein is the use of variants of said *Ng*NOA, which may have a sequence substantially identical to SEQ ID NO:4 or at least about 70%, preferably at least about 80%, more preferably at least about 85%, 90% or 95%, even more preferably at least about 96%, 97%, 98% or 99% sequence identity to SEQ ID NO:4.

In particular embodiments, the recombinant nucleic acid encoding the NOA protein has been codon-optimized for expression in one or more microalgae of interest. Accordingly, disclosed herein are recombinant microalgae, in particular microalgae of a *Phaeodactylum* species, which have been transformed with a recombinant nucleic acid comprising SEQ ID NO:5, which sequence encodes the same protein as the sequence of SEQ ID NO:3, but wherein the coding sequence was codon-optimized for expression in a *Phaeodactylum* species.

Also envisaged herein are variants or homologs of the proteins and enzymes involved in an NO production pathway as described herein. It is understood that the variant proteins or enzymes described herein may have conservative or non-essential amino acid substitutions, which do not have a substantial effect on the protein function. Whether or not a particular substitution will be tolerated (i.e., will not adversely affect desired biological properties) can be determined as described in Bowie et al. (1990) (Science 247:1306 1310). A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine), and aromatic side chains (e.g., tyrosine, phenylalanine, tryptophan, histidine).

Additional protein and enzyme variants are those in which additional amino acids are fused to the enzyme, such as restriction sites for cloning purposes.

The recombinant micro-organisms described herein may comprise an exogenous nucleic acid encoding a protein or an enzyme involved in an NO production pathway, and/or may over-express an endogenous nucleic acid encoding a protein or an enzyme involved in an NO production pathway.

Methods for increasing expression of an endogenous nucleic acid are known in the art and include but are not limited to introducing one or more copies of the endogenously present nucleic acid, optionally under control of stronger promoters, introducing transcription activators, capable of activating transcription of the endogenous gene etc.

The methods described herein envisage genetically engineering micro-organisms, in particular microalgae, for producing an increased amount of certain molecules of interest.

Accordingly, the herein described methods aim to increase the production of molecules of interest, in particular molecules of the lipid metabolic pathway, including lipids as well as intermediates of said lipid metabolic pathway such as fatty acids. In addition, the herein described methods may also increase the production of biomolecules derived from said molecules of the lipid metabolic pathway such as hydrocarbons, fatty alcohols, etc. In particular, the production of lipids, more particularly TAGs, is increased.

In particular embodiments, the micro-organisms described herein have further been genetically modified to ensure production of molecules of interest, in particular molecules of the lipid metabolic pathway or biomolecules derived from said molecules. Further genetic modifications to further increase lipid production, in particular TAG production, as well as the production of any intermediate of the lipid metabolic pathway, are envisaged herein, but also further genetic modifications to ensure (increased) production of a biomolecule of interest derived from a molecule of the lipid metabolic pathway. For example, the recombinant micro-organisms described herein may be further genetically modified to further increase fatty acid biosynthesis and/or TAG assembly. In other examples, the recombinant micro-organisms may be further genetically modified to ensure production of e.g. hydrocarbons from fatty acids, or fatty alcohols from acetyl-CoA.

As metabolic pathways are well-established in micro-organisms, methods for modifying the lipid metabolic pathway and the production of biomolecules derived from molecules of said lipid metabolic pathway in a micro-organism as described herein can be easily determined by the skilled person, including in microalgae. Standard reference work setting forth the general principles of biochemistry includes "Biochemical Pathways: An Atlas of Biochemistry and Molecular Biology", ed. Michal, G, John Wiley and Sons, Inc., New York, US, 1999. In the field of microalgae, standard reference work includes: "Handbook of Microalgal Culture: Applied Phycology and Biotechnology", 2nd Edition, Amos Richmond and Qiang Hu, WileyBlackwell, 2013.

The methods described herein envisage transforming micro-organisms, in particular microalgae, to stimulate the lipid metabolic pathway and/or to increase production of biomolecules derived from molecules of the lipid metabolic pathway. Accordingly, the methods encompass providing a microbial strain suitable for lipid production or wherein the lipid production is to be increased. Such a strain is preferably a microbial strain which produces lipids.

In the method of the invention, the micro-organism is a microalga. Preferably, the microalga is selected from the Chromalveolata, more preferably the Heterokontophyta, even more preferably the Bacillariophyceae (diatoms), including the Naviculales such as *Phaeodactylum tricornutum* and the Pennales (pennate diatoms), and/or the Eustigmatophyceae, including *Nannochloropsis* species such as *Nannochloropsis gaditana.* In particular embodiments, the microalga is *Phaeodactylum tricornutum,* including the Pt1 strain.

In further particular embodiments, the recombinant microalga is *Phaeodactylum tricornutum* which has been modified to ensure an increased NO production by transformation with one or more recombinant nucleic acids encoding a NOA protein such as one or more of *Pt*NOA, *Ng*NOA or a variant thereof.

Most microalgae are photoautotrophs, i.e. their growth is strictly dependent on the generation of photosynthetically-derived energy. Their cultivation hence requires a relatively controlled environment with a large input of light energy. For certain industrial applications, it is advantageous to use heterotrophic microalgae, which can be grown in conventional fermenters. In particular embodiments the microalgae have been further metabolically engineered to grow heterotrophically (i.e. to utilize exogenous organic compounds (such as glucose, acetate, etc.) as an energy or carbon source). A method for metabolically engineering microalgae to grow heterotrophically has been described in US Patent No. 7,939,710. In particular embodiments, the microalgae are further genetically engineered to comprise a recombinant nucleic acid encoding a glucose transporter, preferably a glucose transporter selected from the group consisting of Glut 1 (human erythrocyte glucose transporter 1) and Hup1 (Chlorella HUP1 Monosaccharide-H+ Symporter). The glucose transporters facilitate the uptake of glucose by the host cell, allowing the cells to metabolize exogenous organic carbon and to grow independent of light. This is particularly advantageous for obligate phototrophic microalgae. Lists of phototrophs may be found in a review by Droop (1974. Heterotrophy of Carbon. In Algal Physiology and Biochemistry, Botanical Monographs, 10:530-559, ed. Stewart, University of California Press, Berkeley), and include, for example but without limitation, organisms of the phyla Cyanophyta (Blue-green algae), including the species *Spirulina* and *Anabaena*; Chlorophyta (Green algae), including the species *Dunaliella, Chlamydomonas,* and *Heamatococcus;* Rhodophyta (Red algae), including the species *Porphyridium, Porphyra, Euchema,* and *Graciliaria;* Phaeophyta (Brown algae), including the species, *Macrocystis, Laminaria, Undaria,* and *Fucus;* Baccilariophyta (Diatoms), including the species *Nitzschia, Navicula, Thalassiosira,* and *Phaeodactylum;* Dinophyta (Dinoflagellates), including the species *Gonyaulax;* Chrysophyta (Golden algae), including the species Irsochrysis and *Nannochloropsis*; Cryptophyla, including the species *Cryptomonas*; and Euglenophyta, including the species *Euglena.*

In the methods of the invention, the recombinant microalga are cultured under conditions suitable for the production of NO by the recombinant microalgae so as to increase production of TAG. More particularly this implies "conditions sufficient to allow expression" of the recombinant nucleic acid (encoding a NOA protein). Typically the culture conditions are also selected so as to favor production of molecules of interest, in particular molecules of the lipid metabolic pathway or biomolecules derived from said molecules.

Culture conditions can comprise many parameters, such as temperature ranges, levels of aeration, and media composition. Each of these conditions, individually and in combination, allows the micro-organism to grow. To determine if conditions are sufficient to allow (over)expression, a micro-organism can be cultured, for example, for about 4, 8, 12, 18, 24, 36, or 48 hours. During and/or after culturing, samples can be obtained and analyzed to determine if the conditions allow (over)expression. For example, the micro-organisms in the sample or the culture medium in which the micro-organisms were grown can be tested for the presence of a desired product, e.g. NO or a transcript of the recombinant nucleic acid. When testing for the presence a desired product, assays, such as, but not limited to, polymerase chain reaction (PCR), sodium dodecyl sulfate polyacrylamide gel electrophoresis (SDS-PAGE), TLC, HPLC, GC/FID, GC/MS, LC/MS, MS, can be used. In particular, when testing for the presence of NO, the fluorophore 4-amino-5-methylamino-2',7'-difluororescein diacetate (DAF-FM) can be used as described elsewhere herein.

Exemplary culture media include broths or gels. The micro-organisms may be grown in a culture medium comprising a carbon source to be used for growth of the micro-organisms. Exemplary carbon sources include carbohydrates, such as glucose, fructose, cellulose, or the like, that can be directly metabolized by the host cell. In addition, enzymes can be added to the culture medium to facilitate the mobilization (e.g., the depolymerization of starch or cellulose to fermentable sugars) and subsequent metabolism of the carbon source. A culture medium may optionally contain further nutrients as required by the particular strain, including inorganic nitrogen or phosphorous sources, and the like, and minerals and the like. In particular embodiments, wherein phototrophic microalgae are used, the method for increasing the production of TAG, may comprise providing recombinant microalgae genetically engineered to produce or overproduce NO by transformation with a recombinant nucleic acid encoding a NOA protein, and culturing said microalgae in photobioreactors or an open pond system using CO₂ and sunlight as feedstock.

Other growth conditions, such as temperature, cell density, and the like are generally selected to provide an economical process. Temperatures during each of the growth phase and the production phase may range from above the freezing temperature of the medium to about 50°C.

The culturing step of the methods described herein can be conducted continuously, batchwise, or some combination thereof.

The culturing conditions which are suitable for the production of NO by the recombinant microalgae are also suitable for growth of the recombinant microalga, such that the production of TAGs is concomitant with the growth of the microalga. In other words, the cell concentration is not substantially impacted by modulating the NO production pathway, in particular by NOA overexpression, in the genetically engineered strains, such that the production of TAGs can occur during the growth phase of the microalga, which can increase improve overall productivity of TAG.

In particular embodiments, the methods for the increased production of TAGs, further comprise the step of recovering said TAGs from the recombinant micro-organisms and/or from the cultivation medium. Accordingly, in particular embodiments the methods comprise recovering TAGs, or biomolecules derived from said TAGs as envisaged herein, from the recombinant micro-organism and/or from the cultivation medium.

Methods for the recovery of said molecules or biomolecules from microalgae are known in the art and typically involve cell disruption and extraction of the molecules of interest (Guldhe et al. 2014, Fuel 128:46-52). Alternatively or in addition, the recombinant microalgae may be further genetically engineered to ensure secretion of the molecules of interest. A further example includes a hydrothermal processing (HTL) of microalgae to produce biocrude, from which the molecules of interest can be recovered or which can be further processed to e.g. biofuel.

As noted before, the present inventors have surprisingly found that culturing the recombinant microalgae according to the invention results in increased TAG production.

In particular, the recombinant microalgae ensure a rate of TAG production, which is sufficiently high to be industrially valuable. More particularly, the TAG content in said recombinant microalgae is at least 150% 160%, 170%, 180% or 190%, even more preferably at least 200% or more as compared to microalgae wherein the NO production was not modulated such as a wild-type microalgae or microalgae that have been transformed with an empty vector. In other words, the present method causes an increase in the TAG content of the recombinant microalga compared to a microalga wherein the NO production was not modulated by a factor of pat least 1.5, 1.6, 1.7, 1.8 or 1.9, even more preferably at least 2 or even beyond.

The lipid content, in particular the TAG content, of a micro-organism can be measured using a variety of methods well known in the art. Non-limiting examples include staining with the fluorophore Nile Red (excitation wavelength at 485 nm; emission at 525 nm) and measurement of Nile Red fluorescence, and mass spectrometry (MS).

In particular embodiments, the methods include optimizing said microalgae and/or the cultivation medium so as to ensure the production of TAG. This can encompass modifying the microalga so as to further the production and/or preventing the catabolism of TAG, for instance by blocking other biosynthesis pathways. For instance, where production of TAG is envisaged, the methods of the present invention may additionally comprise modifying the microalga of interest by blocking of pathways that divert carbon to alternative metabolic routes and/or preventing TAG catabolism. Such methods have been described in the art, as noted in the background section herein. Additionally or alternatively the cultivation or production conditions can be adjusted to stimulate the production of TAG. In particular embodiments, the microalgae are modified to block non-desirable pathways, such as programmed cell death. In particular embodiments, the methods of the invention comprise maintaining the cell under conditions which ensure that cell viability is maintained or is at an acceptable level.

The recombinant micro-organisms and microalgae described herein may hence be particularly suitable for industrial applications such as biofuel production and the production of biomolecules e.g. for chemical applications, for use in food industry, for the production of cosmetics, etc. Hence, further disclosed herein is the use of the recombinant micro-organisms and microalgae described herein for biofuel production or production of biomolecules (e.g. fatty acids), e.g. for chemical industry, for food industry, for cosmetics, etc.

Also disclosed herein are methods for obtaining a recombinant micro-organism such as a recombinant microalga capable of (over)producing NO as described herein. Such methods may comprise ensuring (over)expression of a gene encoding a protein involved in NO production. In particular, the methods may comprise transforming a micro-organism with a recombinant nucleic acid encoding a protein involved in an NO production pathway as taught herein. In particular, the method may comprise the steps of: a) transforming a micro-organism with a recombinant nucleic acid encoding a protein involved in an NO production pathway as described herein above; and b) selecting a micro-organism capable of (over)producing NO. As detailed above the recombinant nucleic acid may encode a protein which is endogenous or foreign to the micro-organism. Additionally or alternatively the methods may involve modifications which induce or increase endogenous expression of a gene encoding a protein involved in NO production as described above.

The methods for generating the recombinant micro-organisms described herein involve standard genetic modifications, for which well-established methods are available to the skilled person.

More particularly, genetic engineering of the micro-organisms containing a recombinant nucleic acid encoding a protein involved in an NO production pathway as described herein may be accomplished in one or more steps via the design and construction of appropriate vectors and transformation of the micro-organisms with those vectors.

Methods for transforming microalgae are well known to a skilled person. For example, electroporation and/or chemical (such as calcium chloride- or lithium acetate-based) transformation methods or *Agrobacterium* tumefaciens-mediated transformation methods as known in the art can be used.

Numerous vectors are known to practitioners skilled in the art and any such vector may be used. Selection of an appropriate vector is a matter of choice. Preferred vectors are vectors developed for microalgae such as the vectors called pH4-GUS, pCT2, and pCT2Ng.

Typically, a vector may comprise a recombinant nucleic acid encoding a protein involved in an NO production pathway as described herein. In particular, a vector may comprise the coding sequence of a protein involved in an NO production pathway as described herein and associated promoter and terminator sequences. The vector may further contain restriction sites of various types for linearization or fragmentation. The vector may further include an origin of replication that is required for maintenance and/or replication in a specific cell type. The vector also preferably contains one or more selection marker gene cassettes. A selectable marker gene cassette typically includes a promoter and transcription terminator sequence, operatively linked to a selectable marker gene. Suitable markers may be selected from markers that confer antibiotic resistance, herbicide resistance, visual markers, or markers that complement auxotrophic deficiencies of a host cell, in particular a microalga. For example, the selection marker may confer resistance to an antibiotic such as hygromycin B (such as the hph gene), zeocin/phleomycin (such as the ble gene), kanamycin or G418 (such as the nptll or aphVIII genes), spectinomycin (such as the aadA gene), neomycin (such as the aphVIII gene), blasticidin (such as the bsd gene), nourseothricin (such as the natR gene), puromycin (such as pac gene) and paromomycin (such as the aphVIII gene). In other examples, the selection marker may confer resistance to a herbicide such as glyphosate (such as GAT gene), oxyfluorfen (such as protox/PPO gene) and norflurazon (such as PDS gene). Visual markers may also be used and include for example beta-glucuronidase (GUS), luciferase and fluorescent proteins such as Green Fluorescent Protein (GFP), Yellow Fluorescent protein, etc. Two prominent examples of auxotrophic deficiencies are the amino acid leucine deficiency (e.g. LEU2 gene) or uracil deficiency (e.g. URA3 gene). Cells that are orotidine-5'-phosphate decarboxylase negative (ura3-) cannot grow on media lacking uracil. Thus a functional URA3 gene can be used as a selection marker on a host cell having a uracil deficiency, and successful transformants can be selected on a medium lacking uracil. Only cells transformed with the functional URA3 gene are able to synthesize uracil and grow on such medium. If the wild-type strain does not have a uracil deficiency, an auxotrophic mutant having the deficiency must be made in order to use URA3 as a selection marker for the strain. Methods for accomplishing this are well known in the art.

Successful transformants can be selected for in known manner, by taking advantage of the attributes contributed by the marker gene, or by other characteristics (such as the ability to produce NO) contributed by the inserted recombinant nucleic acid. Screening can also be performed by PCR or Southern analysis to confirm that the desired insertions have taken place, to confirm copy number and to identify the point of integration of coding sequences into the host genome. Activity (such as NO-producing activity) of the protein encoded by the inserted coding sequence can be confirmed using known assay methods. For example, NO levels can be measured using the fluorophore 4-amino-5-methylamino-2',7'-difluororescein diacetate (DAF-FM), which allows detection of nitric oxide (ONOO⁻) which is in equilibrium with NO (St Laurent et al. 2015 Methods Mol Biol. 1220:339-345).

Methods for modifying endogenous gene expression by the use of artificial transcription factors (ATFs) or activator domains have also been described (Sera T. 2009, Adv Drug Deliv Rev 61:513-526; Maeder et al. 2013, Nat Methods 10:243-245; Cheng et al. 2013, Cell Res 23:1163-1171).

Also disclosed herein are the recombinant micro-organisms, in particular the recombinant microalgae, described herein. These micro-organisms are characterized in that they are genetically engineered to (over)produce NO as described herein and may further be characterized by their increased lipid content. In particular, the TAG content of the recombinant micro-organisms or microalgae described herein is increased to at least 110%, preferably at least 120%, more preferably at least 150% 160%, 170%, 180% or 190%, even more preferably at least 200% as compared to micro-organisms or microalgae wherein the NO production was not modulated such as a wild-type micro-organisms or microalgae or a micro-organism or microalgae that has been transformed with an empty vector.

In particular, the micro-organisms are characterized by the presence of one or more genetic modifications in the genome which affect NO synthesis, as detailed above.

The present invention will now be further illustrated by means of the following non-limiting examples.

### EXAMPLES

### Example 1: Effect of overexpression of the endogenous NOA gene on triacylglycerol production in Phaeodactylum tricornutum

### Material and methods

*Phaeodactylum tricornutum* (Pt1) Bohlin Strain 8.6 CCMP2561 (Culture Collection of Marine Phytoplankton, now known as NCMA: National Center for Marine Algae and Microbiota) was used in example 1.

### Genetic construct for PtNOA overexpression.

Genomic DNA was extracted from *Phaeodactylum tricornutum* Pt1 strain using the following procedure: 100.10⁶ cells were harvested and frozen in liquid nitrogen. A volume of 20 µl Edward-Buffer (Tris-HCI 200 mM, pH 7.5; NaCl 250 mM; EDTA 25 mM; SDS 0.5%, w/v) was added, then samples were homogenized and debris removed by centrifugation. The supernatant was transferred to the same volume of isopropanol to precipitate DNA. After an additional 15 minute centrifugation at 10,000 x g, the pellet was washed with ethanol 70%, dried and solubilized in TE buffer (10 mM Tris-HCL pH7, 1 mM EDTA). DNA concentration was measured using a Nanodrop 2000 spectrophotometer (Thermo Scientific). Using genomic DNA as matrix, a 2352-bp sequence was amplified by polymerase chain reaction (PCR) with the following oligonucleotides designed from Phatr2_56150 (Vardi et al. 2008), and carrying respectively *Xba*I and *EcoR*I restriction sites (underlined sequence): NOA-Fw *Xba*I 5'-TTTATCTAGA**ATG**GTCCCCACTGGTTGTATG-3' (SEQ ID NO:8), NOA-Rev *EcoR*I 5'-TTTAGAATTC**CTA**ATTACGCCCTACACCTTTTCTTC-3' (SEQ ID NO:9). PCR was performed using Phusion High Fidelity polymerase (Thermo Scientific) according to the manufacturer's instructions. PCR product was digested by *EcoR*I and *Xba*I*,* purified and cloned in the linearized expression vector. The expression vector used for overexpression corresponds to the pH4-GUS vector (De Riso et al. 2009 Nucleic Acids Res. doi: 10.1093/nar/gkp448). The vector contains a gene coding for resistance to zeocin (Shble), allowing selection of transformed cells. Expression of the NOA gene is controlled by the constitutive histone 4 promoter (H4pro). The vector sequence is provided in SEQ ID NO:6 (Fig. 2B) and the vector map is shown in Figure 2A.

### Transformation of P. tricornutum with the genetic construct and selection of strains overexpressing NOA

Wild-type P. *tricornutum* cells were transformed via particle-bombardment under aseptic conditions according to Russel Kikkert et al. (1993) (The Biolistic® PDS-1000/He device, Plant Cell, Tissue and Organ Culture, Volume 33, Issue 3, pp 221-226). A modified diatom protocol (Falciatore et al. 1999. Mar Biotechnol (NY) 1:239-251) was used. Briefly, three to four days-old Pt1 cultures were concentrated to 4.10⁷ cells.500 µl⁻¹ and spread onto a 1 % agar-plate containing artificial seawater (ESAW) medium (Table 1) with 50% reduced concentration of salt solution 1 and 2 (see Table 1 for the composition of salt solution 1 and 2). While vortexing, 2-3 µg of non-linearized plasmid were added to 25 µl ethanol-sterilized tungsten particles (SIGMA), together with 25 µl of 2.5 M CaCl₂ and 10 µl of 0.1 M spermidine. The mix was vortexed for three minutes, pelleted and washed two times (full speed, 5 s, room temperature) with 700 µl ethanol. Finally, DNA-coated tungsten particles were re-suspended in 25 µl ethanol. 12 µl of the mix were transferred onto a macrocarrier and the bombardment was carried out using 1,550 psi rupture disks (BioRad). After two to three days of incubation under continuous illumination, cells were transferred to the same kind of agar-plates containing 100 µg.ml⁻¹ zeocin (Promega) for the selection of resistant transformants (i.e. genetically modified strains). Colonies appearing 4 to 6 weeks afterwards were transferred to a new plate for one week, prior to the inoculation of 20 ml liquid cultures.

### Culture of P. tricornutum at different scales, different media, different CO₂ supplies and different illumination regimes.

For batch cultures, 20 ml or 50 ml cultures were grown in 250 ml Erlenmeyer flasks at 20°C in ESAW medium. Cells were grown on a 12:12 light (30 µE m⁻².sec⁻¹) / dark cycle.

For experiments in small photobioreactors, cells were pre-cultured in 250 ml Erlenmeyer flasks until they reached a density of 2-4.10⁶ cells/ml. Cells were then centrifuged at 3,500 g for 5 minutes and re-suspended in either 10 x ESAW medium (containing ten times more N and P; Table 1), medium E (Anandarajah et al. 2012 Applied Energy 96: 371-377; Table 2) or medium F (Benvenuti et al. J Appl Phycol 27:1425-1431; Table 3) to a final concentration of 2.10⁶ cells/ml. These two additional media were chosen from the published literature because of their very different macronutrients and microelements composition, thus testing TAG accumulation in NOA overexpressor(s) across culture conditions. Cells were grown under a constant light regime at 20°C in small scale bioreactors (Multi-Cultivator MC 1000, Photon Systems Instruments, Czech Republic), where temperature and light are tightly controlled. Culture mixing throughout cultivation time was provided by gas sparging as in air-lift photobioreactors; in order to test precise CO₂ supplies to bioreactor tubes, the Gas Mixing System GMS 150 (Photon Systems Instruments, Czech Republic) was used following manufacturer's instructions.

**Table 1: Composition of ESAW 1X culture medium. Salt Solution 1 is autoclaved separately and added aseptically to the final medium. NaNO₃ and NaH₃PO₄ are added in 10 x concentration or left absent.**

| **Salt Solution 1** | **10 x Stock Concentration** | | | | **Final medium concentration** |
|---|---|---|---|---|---|
| NaCl | 211.94 g/L | | | 100 ml per 1 L final medium | 21.194g/L |
| Na₂SO₄ | 35.50 g/L | | | | 3.550 g/L |
| KCI | 5.99 g/L | | | | 0.599 g/L |
| NaHCO₃ | 1.74 g/L | | | | 0.174 g/L |
| KBr | 0.863 g/L | | | | 0.0863 g/L |
| H₃BO₃ | 0.230 g/L | | | | 0.0230 g/L |
| NaF | 0.028 g/L | | | | 0.0028 q/L |
| **Salt Solution 2** | | | | | |
| MgCl₂•6H₂O | 95.92 q/L | | | 100 ml per 1 L final medium | 9.592 g/L |
| CaCl₂•2H₂O | 13.44 g/L | | | | 1.344 g/L |
| SrCl₂•6H₂O | 0.218 g/L | | | | 0.0218 q/L |
| **Major Nutrients** | | | | | |
| NaNO₃ | 46.67 g/L | | | 1 ml per 1 L final medium (or 10 ml/L 10X ESAW) | 46.67 mg/L |
| NaH₂PO₄ | 3.094 g/L | | | 1 ml per 1 L final medium (or 10 ml/L 10X ESAW) | 3.094 mg/L |
| **TRACE METALS** | **stock** | **For 50 ml Trace Metal solution** | | | |
| Na₂EDTA•2H₂O | 3.09 g/100 mL | 5 ml | | 1 ml Trace Metal solution per 1 L final medium | 3.09 mg/L |
| ZnSO₄•7H₂O | 0.73 g/100 ml | 0.5 ml | | | 73µg/L |
| COCl₂•6H₂O | 0.16 g/100 ml | 0.5 ml | | | 16 µg/L |
| MnCl₂•4H₂O | 0.54 g/100 mL | 5 ml | | | 540 µg/l |
| Na₂MoO₄•2H₂O | 0.148 g/100 ml | 50µl | | | 1,48µg/l |
| Na₂SeO₃ | 0.017 g/100 mL | 50µl | | | 0,173 µg/l |
| NiCl₂•6H₂O | 0.149 g/100 mL | 50µl | | | 1,49 µg/l |
| CuSO₄•5H₂O | 0.98 g/100 mL | 50µl | | | 9,8 µg/l |
| **Other** | **Stock Concentration** | | | | |
| EDTA-Iron | 3g/L | | 1 ml per 1 L final medium | 3 mg/L | |
| | | | | 1 mg/L Vitamin H | |
| Vitamin stock | | | 1 ml per 1 L final medium | 1 mg/L Vitamin B12 | |
| | | | | 0.2 g/L Vitamin B1 | |
| **Final Medium Prep:** | **For 1L** | | **For 1L solid:** | **ESAW1X** | **ESAW 0,5X** |
| Ingredient Added before autoclave | Volume | | Ingredient Added before autoclave | Volume | Volume |
| Salt Solution 2 | 100 ml | | H₂O | 800ml | 800ml |
| NaH₂PO₄ | 1ml (or 10 ml/L 10X ESAW) | | Agar 1% (w/v) | 10g | 10g |
| Trace metals stock | 1ml | | Ingredient Added after autoclave | Volume | Volume |
| EDTA-Iron | 1ml | | Sea Salt1 | 100ml/L | 50ml/L |
| Water | up to 0,9L | | Sea Salt2 | 100ml/L | 50ml/L |
| Ingredient Added after autoclave | Volume | | NaNO₃ | 1ml/l | 1ml/l |
| Salt Solution 1 | 100ml | | NaH₂PO₄ | 1ml/l | 1ml/l |
| NaNO₃ | 1ml (or 10 ml/L 10X ESAW) | | Trace metals stock | 1ml/l | 1ml/l |
| Vitamins | 1ml | | EDTA-Iron | 1ml/l | 1ml/l |
| | | | Vitamins | 1ml/l | 1ml/l |

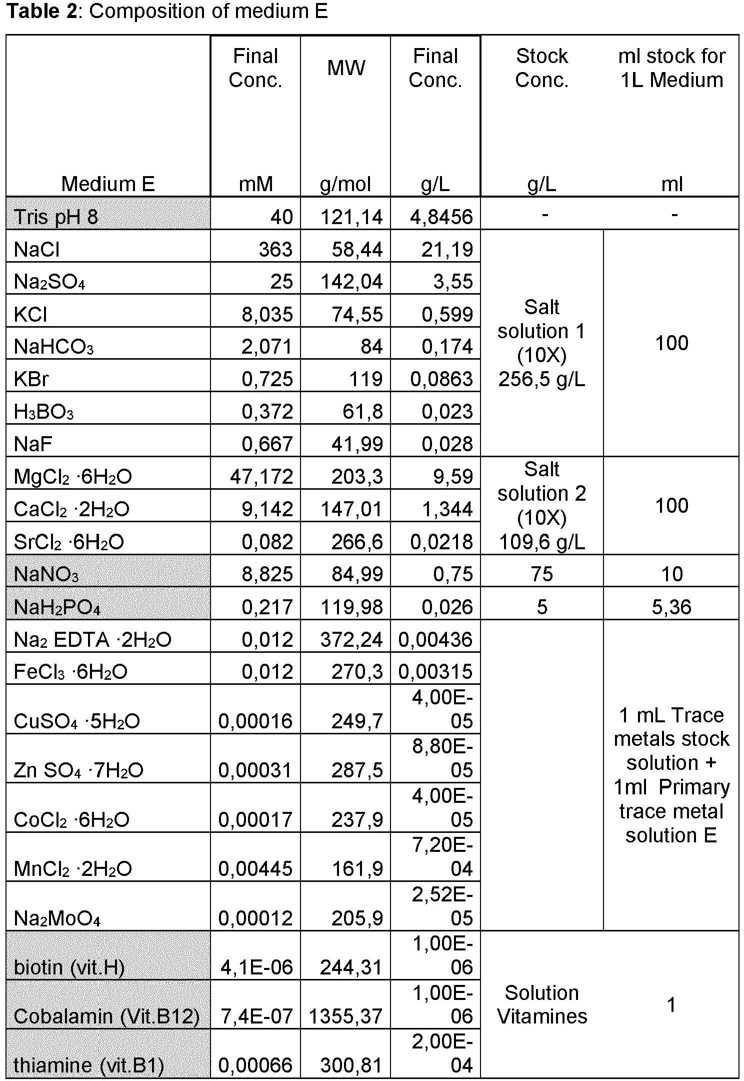

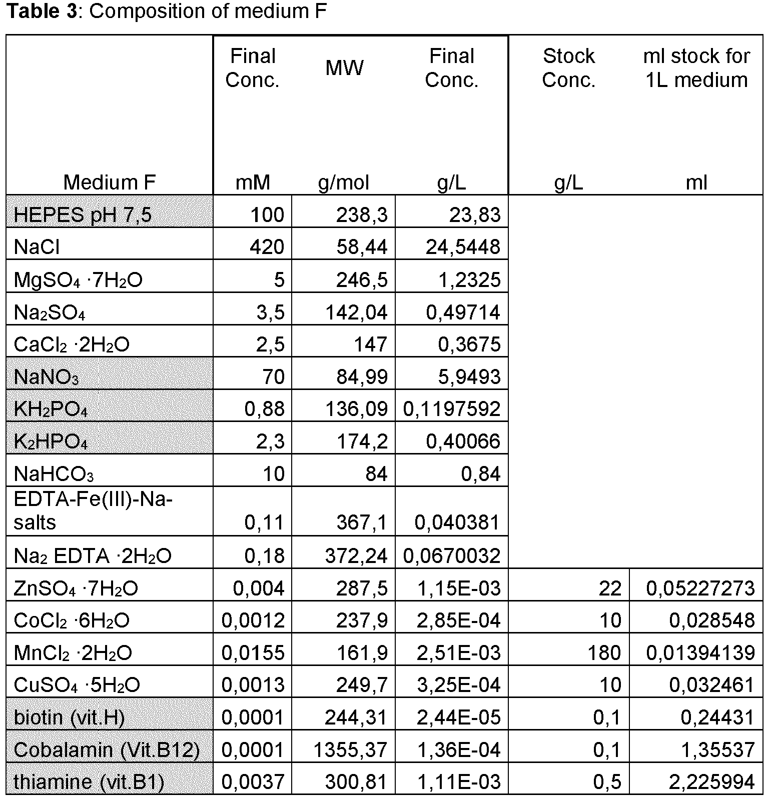

### Measurement of NOA gene expression

To quantify the NOA mRNA level in the genetically modified strains, quantitative polymerase chain reaction (qPCR) was performed after reverse transcription (RT) of extracted RNA. RNA was extracted from 10⁷ cells that were previously pelleted, frozen in liquid nitrogen and stored at -80°C until processing. A volume of 1 ml TriReagent^{®} (SIGMA) was added to the frozen pellet and transferred to a new Eppendorf tube. After vortexing for 30 seconds, samples were incubated for 5 min at room temperature. 200 µl chloroform were added and tubes inverted and incubated for 15 min at room temperature. Phase separation was achieved by centrifugation (30 min, full speed, 4°C). The upper phase was transferred to a new tube and RNA precipitated using 1 volume isopropanol (30 min, full speed, 4°C), washed with 75 % ice cold ethanol (5 min, full speed, 4°C) and the pellet was dried in a Speed Vac system (Eppendorf Concentrator 5301) prior to resuspension in 30 µl DECP water (SIGMA) at 65°C for 10 min. RNA was purified following a second ethanol precipitation using 1 volume of 5 M NH₄⁺, acetate (2.5 M final concentration) and 1 volume isopropanol. Samples were incubated for 10 min on ice and centrifuged, washed, dried and re-suspended as described above. Concentration was determined using a NanoDrop device (Life Inc.). 1000 ng RNA were used for reverse transcription after DNAse treatment (QIAGEN) following manufacturer's instructions so as to yield 1000 ng cDNA, which were diluted to 10 ng.µl⁻¹. For quantitative real time PCR, the housekeeping gene oligonucleotides described by Siaut et al. (2007 Gene 406(1-2):23-35), namely 30S Ribosomal Protein Subunit (RPS) (5'-CGAAGTCAACCAGGAAACCAA-3' (SEQ ID NO:10) and 5'-GTGCAAGAGACCGGACATACC-3' (SEQ ID NO:11)) and tubulin A (TubA) (5'-CTGGGAGCTTTACTGCTTGGA-3' (SEQ ID NO:12) and 5'-ATGGCTCGAGATCGACGTAAA-3' (SEQ ID NO:13)), were used as internal controls. NOA-binding oligonucleotides were 5'-CCTGAAAAGTTCGCTACGCA-3' (SEQ ID NO:14) and 5'-CGGATCCTTTTTGCCCTGAG-3' (SEQ ID NO:15). The total qPCR reaction volume was 10 µl (120 nM per oligonucleotide, 20 ng cDNA, 5 µl 2X SYBR Green Sso Advanced (BioRad). A two-step thermo-profile in 40 cycles was applied after 3 min at 95 °C initial denaturation (95 °C 10 sec, 58 °C 30 sec) and a melt curve was detected (from 65°Cto 95 °C with a 0.5 °C increment) (BioRad CFX Connect Real-Time System). Evaluation of gene expression in 3 biological replicates each in technical triplicates were carried out using the CFX Connect Real-Time System software using TubA and RPS as internal controls.

### Measurement of nitric oxide using a fluorescent reporter

NO production was monitored using the fluorophore 4-amino-5-methylamino-2',7'-difluororescein diacetate (DAF-FM), which allows the sensitive detection of low levels of nitric peroxide (ONOO-) ,which is in equilibrium with NO and thus indicates NO levels (St Laurent et al. 2015. Methods Mol Biol. 1220:339-45) and was previously used to detect NO levels in *P. tricornutum* cells (Vardi et al., 2008). 10 ml culture were diluted to 10⁶ cells/ml and cells were incubated with 20 µl 5 mM DAF-FM (1.5 h, room temperature, darkness, shaking). Cells were washed and re-suspended in 10 ml 10 x ESAW media and aliquoted to 500 µl cultures on a 48 well culture plate. For the examination of DAF-FM-dependent detection of nitric peroxide, 150 µl of the culture were transferred into a 96 well plate and fluorescence was measured with a TECAN infinite M1000Pro plate reader (excitation wavelength at 488 nm, emission at 529 nm).

### Measurement of TAG accumulation by Nile Red staining

A first method to measure the accumulation of TAG droplets consisted in their detection by Nile Red (Sigma Aldrich) fluorescent staining (Excitation wavelength at 485 nm; emission at 525 nm) as previously described (Abida et al., 2015). In brief, cells were diluted and adjusted to a cell density that was linearly correlated with Nile Red fluorescence. Nile Red solution (40 µl of 2.5 µg/mL stock concentration, in 100% DMSO) was added to 160 µl cell suspension. Oil bodies stained with Nile Red were then visualized using a Zeiss AxioScope.A1 microscope (FITC filter; Excitation wavelength at 488 nm; emission at 519 nm). The productivity, corresponding to the accumulation of TAG per volume and per time unit was calculated based on the staining by Nile Red, and expressed in relative fluorescence unit (Rfu) of Nile Red per mL and per day of incubation. Alternatively, Nile red fluorescence values were normalized to the cell concentration.

### Measurement of TAG accumulation by Mass spectrometry

Glycerolipids were extracted from freeze-dried P. *tricornutum* cells grown in 50 mL of medium. About 50 to 100.10⁶ cells are required for a triplicate analysis of TAGs. First, cells were harvested by centrifugation, then immediately frozen in liquid nitrogen. Once freeze-dried, the pellet was suspended in 4 mL of boiling ethanol for 5 minutes to prevent lipid degradation, and lipids were extracted as described by Simionato et al., (2013 Eukaryot Cell. 201, 12(5):665-76) by addition of 2 mL methanol and 8 mL chloroform at room temperature. The mixture was then saturated with argon and stirred for 1 hour at room temperature. After filtration through glass wool, cell debris was rinsed with 3 mL chloroform/methanol 2:1, v/v, and 5 mL of NaCl 1% were then added to the filtrate to initiate biphase formation. The chloroform phase was dried under argon before solubilizing the lipid extract in 1 ml of chloroform. Total glycerolipids were quantified from their fatty acids, in a 10 µl aliquot fraction a known quantity of 15:0 was added and the fatty acids present were transformed as methyl esters (FAME) by a 1 hour incubation in 3 mL 2.5% H₂SO₄ in pure methanol at 100°C (Jouhet et al. 2003 FEBS Lett. 544(1-3):63-8). The reaction was stopped by addition of 3 mL water, and 3 mL hexane were added for phase separation. After 20 min of incubation, the hexane phase was transferred to a new tube. FAMEs were extracted a second time via the addition, incubation and extraction of another 3 ml hexane. The combined 6 ml were argon-dried and re-suspended in 30 µl hexane for gas chromatography-flame ionization detector (GC-FID) (Perkin Elmer) analysis on a BPX70 (SGE) column. FAME were identified by comparison of their retention times with those of standards (Sigma) and quantified by the surface peak method using 15:0 for calibration. Extraction and quantification were performed with at least three biological replicates. TAGs were analyzed and quantified by HPLC-MS/MS. For a technical triplicate analysis, an aliquot of the lipid extract containing 25 nmol of total fatty acid was dried under argon and dissolved in 100 µl of a methanol/chloroform solution (1:2) containing 125 pmol of 18:0/18:0/18:0 TAG as internal standard. For each replicate, 20 µl were injected in the HPLC-MS/MS system. The analytic device comprised a LC system with binary pumps (Agilent 1260 Infinity) coupled to a QQQ MS (Agilent 6460) equipped with a JetStream electrospray vane of injection. TAGs were separated by HPLC from other lipids using a diol column (Macherey-Nagel, EC 150/2 Nucleosil 100-5 OH) maintained at 40°C. The chromatography conditions were as follows: solvent A: isopropanol / water / ammonium acetate 1 M pH 5.3 (850 / 125 / 1); solvent B: Hexane / isopropanol / water / ammonium acetate 1 M pH 5.3 (625 / 350 / 24 / 1); gradient: 0 to 5 min 100% B, 5 to 30 min linear increase of A to 100%, 30 to 45 min 100% A, 45 to 50 min : linear increase of B to 100%, 50 to 70 min 100% B. Under these conditions, TAGs were eluted after 4 - 5 min of run. The various TAG species were detected from their m/z ratio by MS/MS using the Multiple Reaction Monitoring (MRM) mode. The various transition reactions used to identify the different TAG species are those previously established with Phaeodactylum tricornutum (Abida et al. 2015). Quantification was made using the Agilent Mass Hunter^{®} software furnished by the MS supplier.

### Results

Results shown in Figure 3 show that after transformation of P. *tricornutum* cells with pH4-NOAOE vector, *Pt*NOA is overexpressed. Two exemplary strains are shown in Figure 3, NOAOE4 and NOAOEf, which were obtained by two independent transformation experiments.

The NOA overexpressing strains contained higher levels of NO, as shown by the DAF-FM fluorescent NO-indicator. The endogenous level of NO was higher and accumulated more rapidly than in a wild-type strain (Fig. 4A). After 3 hours of incubation with DAF-FM, increased levels of NO were measured in both NOAOE4 and NOAOEf strains as compared to WT Pt1 cells and Pt1 cells transformed with the pH4-GUS vector (Fig. 4B).

NOA overexpressing strains also contained more TAG per cell (Fig. 5A) and increased TAG productivity (Fig. 5B). Also, as illustrated in Figure 7, the cell concentration of NOA overexpressing strains (NOAOE4 and NOAOEf) was barely or not impacted by the NOA overexpression compared to the strain in which the NO production pathway was not modulated (pH4). The increased productivity of TAG in the P. *tricornutum* cells was observed in cells grown in different media, different volumes, different systems and different supplies of CO₂ compared to WT Pt1 cells and/or Pt1 cells transformed with the pH4-GUS vector described above (Fig.6).

### Example 2: Overexpression of a PtNOA homolog from Nannochloropsis gaditana (NgNOA) in P. tricornutum and N. gaditana

A *Pt*NOA homolog (Fig. 1A) is present in *Nannochloropsis gaditana* (*Ng*NOA; SEQ ID NO:3 and SEQ ID NO:4 for the nucleotide and amino acid sequence, respectively).

For the heterologous expression of *Ng*NOA in *Phaeodactylum tricornutum* and the overexpression in *Nannochloropsis gaditana,* the coding sequence was optimized to match the codon optimization of both Chromalveolata species. Restriction sites for *BamH*I and *Xba*I were added at the 5'-end, and *EcoR*I and *Nde*I at the 3'-end of the codon optimized CDS to allow expression in the pH4 vector for expression in P. *tricornutum,* and in the PCT2Ng vector (SEQ ID NO:7) for the expression in *N. gaditana,* respectively. The codon optimized *Ng*NOA coding sequence is designated as *Ng*NOAoptCDS and has the following sequence:

### SEQUENCE LISTING

<110> TOTAL RAFFINAGE CHIMIE
<120> INCREASED TRIACYLGLYCEROL PRODUCTION IN MICROALGAE
<130> TOTAL-221-PCT
<150> EP16153390.6
   <151> 29-01-2016
<160> 15
<170> Patent In version 3.5
<210> 1
   <211> 2427
   <212> DNA
   <213> Phaeodactylum tricornutum
<400> 1
   atggtcccca ctggttgtat gagatcaaag aacaaacaga gtactgctgc tgacggaaga      60
   aggtcgattg gaagtcgatc caacgctgga ctggtcatgt tctttagtat gactatctta     120
   tcgacacgac gatttgcggc agcttgggtt tttcagagta gtggaataca acgatcaata     180
   ccatccacgc ataggatgcg aacgaatttt gctttgtcga cgcgctgctt tgcttcttca     240
   tccgacaacc atgacgaaga ggaacaacga gactctccga aacaaagatc caaacgcagc     300
   caaactaatc ggtccaagaa attcaaaatt gctgaatcaa tcgaccagag caaaatagat     360
   aagctagcac aagcattcga tgaactcgct cggaaggaag gcttcgactc gtcaacagca     420
   cgctttgccg acgatgtgac gttcgaggac aagtttgacg acgattcgtt tctggacgat     480
   gacgatgata acaacaaaga taaagtggga aacttgcacc tagatgcatc catgttcagt     540
   ttaagtgact ttatagataa gagtgaggaa gatggcggca atccaaccga tcaagatgac     600
   gaggactacc ttgattttgg tgcagacatt gacatgagta tagaagcaag gattgccgct     660
   gccaaacggg atatggatct cggtcgagtc agcgcccctc ccgatatgag atcctcgcgc     720
   agggaggtaa ctgcagccga ccttcgcaaa cttggatttc gaaccgaggc aaacccattc     780
   ggcaacgacg aaactccacg gaaggagcgc ttccagttgg taacaaactc catgtcgtgc     840
   tccgcctgtg gatcggactt tcaatgccac aacgaagatc ggcccggata tctgcctcct     900
   gaaaagttcg ctacgcaaac agcacttgga aaaatagaac agatgcaaaa gttgcaggat     960
   aaagcagaaa aagcggaatg gacacctgaa gatgagattg aatggttgat tcagactcag    1020
   ggcaaaaagg atccgaacaa agaaatgcag gaggtgcccc agatcgatgt tgattctttg    1080
   gcaggggaaa tgggccttga cctcgtagag ctttccaaaa agatggttat ttgcaagcgc    1140
   tgtcacggtc tgcaaaactt tggaaaagtg caagattccc tccgacctgg gtggacgaag    1200
   gagccactgt tgtcgcagga gaaatttcgt gaattgttaa ggccaatcaa ggaaaagccg    1260
   gcagttatcg ttgcattggt cgatcttttt gatttttcgg ggtctgtgct ccctgagctt      1320
   gatgaaatcg ctggtgaaaa ccctgtaatt cttgcggcca acaaggcgga tcttcttcca      1380
   agtgaaatgg gacgcgtgcg agctgagagt tgggttcgac gcgagctcga ataccttgga      1440
   gtcaagtcgt tggccggtat gagaggagca gttcggcttg tcagctgcaa gactggagct      1500
   gggattaatg atttgctgga gaaagcaaga ggattagccg aggaaatcga cggcgacata      1560
   tacgtcgtcg gggctgcaaa tgcaggaaaa agtacgcttt tgaattttgt tctaggtcag      1620
   gacaaggtga acagatcacc cggaaaagca cgagcaggca acaggaatgc cttcaagggc      1680
   gcggtgacga caagtccact gccaggcaca acgcttaagt tcatcaaagt cgatttaggc      1740
   ggcggtcgaa gtctatatga cactcctggt cttctggtat taggcactgt gacacagtta      1800
   ctgacccccg aagagctgaa gatagttgtt cccaaaaagt atgtcaaacc gatcaaactg      1860
   atattcgatt cacagtcaat aatgttcaaa ctaacacctc gttcctcaaa caggccaatt      1920
   gaacctgtca ccctccggct ctctaccgga aagtgcgttc tagttggagg attggcccgc      1980
   atcgagttaa tcggcgactc aagacccttt atgttcacat tttttgttgc taatgagatc      2040
   aagctccacc ctactgacat agagagagcc gatgagttcg ttctaaagca cgctggtggc      2100
   atgttgactc caccgctagc acccggacca aaacgtatgg aagagattgg agaatttgaa      2160
   gatcacatcg tggatatcca gggtgctggc tggaaagaag ctgctgctga tatcagtctt      2220
   accggactag gatgggtggc cgttacagga gcagggacag cgcaagtaaa aataagtgtt      2280
   ccgaaaggta ttggtgtatc ggtgcggcct ccgcttatgc ctttcgatat ctggaaagtt      2340
   gcatcgaagt ataccggaag tcgagctgta agaaagtcat ccaaactggc gaatgggaaa      2400
   cgaagaaaag gtgtagggcg taattag                                          2427
<210> 2
   <211> 783
   <212> PRT
   <213> Phaeodactylum tricornutum
<400> 2
   Met Val Pro Thr Gly Cys Met Arg Ser Lys Asn Lys Gln Ser Thr Ala
   1               5                   10                  15
   Ala Asp Gly Arg Arg Ser Ile Gly Ser Arg Ser Asn Ala Gly Leu Val
               20                  25                  30
   Met Phe Phe Ser Met Thr Ile Leu Ser Thr Arg Arg Phe Ala Ala Ala
           35                  40                  45
   Trp Val Phe Gln Ser Ser Gly Ile Gln Arg Ser Ile Pro Ser Thr His
       50                  55                  60
   Arg Met Arg Thr Asn Phe Ala Leu Ser Thr Arg Cys Phe Ala Ser Ser
   65                  70                  75                  80
   Ser Asp Asn His Asp Glu Glu Glu Gln Arg Asp Ser Pro Lys Gln Arg
                   85                  90                  95
   Ser Lys Arg Ser Gln Thr Asn Arg Ser Lys Lys Phe Lys Ile Ala Glu
               100                 105                 110
   Ser Ile Asp Gln Ser Lys Ile Asp Lys Leu Ala Gln Ala Phe Asp Glu
           115                 120                 125
   Leu Ala Arg Lys Glu Gly Phe Asp Ser Ser Thr Ala Arg Phe Ala Asp
       130                 135                 140
   Asp Val Thr Phe Glu Asp Lys Phe Asp Asp Asp Ser Phe Leu Asp Asp
   145                 150                 155                 160
   Asp Asp Asp Asn Asn Lys Asp Lys Val Gly Asn Leu His Leu Asp Ala
                   165                 170                 175
   Ser Met Phe Ser Leu Ser Asp Phe Ile Asp Lys Ser Glu Glu Asp Gly
               180                 185                 190
   Gly Asn Pro Thr Asp Gln Asp Asp Glu Asp Tyr Leu Asp Phe Gly Ala
           195                 200                 205
   Asp Ile Asp Met Ser Ile Glu Ala Arg Ile Ala Ala Ala Lys Arg Asp
       210                 215                 220
   Met Asp Leu Gly Arg Val Ser Ala Pro Pro Asp Met Arg Ser Ser Arg
   225                 230                 235                 240
   Arg Glu Val Thr Ala Ala Asp Leu Arg Lys Leu Gly Phe Arg Thr Glu
                   245                 250                 255
   Ala Asn Pro Phe Gly Asn Asp Glu Thr Pro Arg Lys Glu Arg Phe Gln
               260                 265                 270
   Leu Val Thr Asn Ser Met Ser Cys Ser Ala Cys Gly Ser Asp Phe Gln
           275                 280                 285
   Cys His Asn Glu Asp Arg Pro Gly Tyr Leu Pro Pro Glu Lys Phe Ala
       290                 295                 300
   Thr Gln Thr Ala Leu Gly Lys Ile Glu Gln Met Gln Lys Leu Gln Asp
   305                 310                 315                 320
   Lys Ala Glu Lys Ala Glu Trp Thr Pro Glu Asp Glu Ile Glu Trp Leu
                   325                 330                 335
   Ile Gln Thr Gln Gly Lys Lys Asp Pro Asn Lys Glu Met Gln Glu Val
               340                 345                 350
   Pro Gln Ile Asp Val Asp Ser Leu Ala Gly Glu Met Gly Leu Asp Leu
           355                 360                 365
   Val Glu Leu Ser Lys Lys Met Val Ile Cys Lys Arg Cys His Gly Leu
       370                 375                 380
   Gln Asn Phe Gly Lys Val Gln Asp Ser Leu Arg Pro Gly Trp Thr Lys
   385                 390                 395                 400
   Glu Pro Leu Leu Ser Gln Glu Lys Phe Arg Glu Leu Leu Arg Pro Ile
                   405                 410                 415
   Lys Glu Lys Pro Ala Val Ile Val Ala Leu Val Asp Leu Phe Asp Phe
               420                 425                 430
   Ser Gly Ser Val Leu Pro Glu Leu Asp Glu Ile Ala Gly Glu Asn Pro
           435                 440                 445
   Val Ile Leu Ala Ala Asn Lys Ala Asp Leu Leu Pro Ser Glu Met Gly
       450                 455                 460
   Arg Val Arg Ala Glu Ser Trp Val Arg Arg Glu Leu Glu Tyr Leu Gly
   465                 470                 475                 480
   Val Lys Ser Leu Ala Gly Met Arg Gly Ala Val Arg Leu Val Ser Cys
                   485                 490                 495
   Lys Thr Gly Ala Gly Ile Asn Asp Leu Leu Glu Lys Ala Arg Gly Leu
               500                 505                 510
   Ala Glu Glu Ile Asp Gly Asp Ile Tyr Val Val Gly Ala Ala Asn Ala
           515                 520                 525
   Gly Lys Ser Thr Leu Leu Asn Phe Val Leu Gly Gln Asp Lys Val Asn
       530                 535                 540
   Arg Ser Pro Gly Lys Ala Arg Ala Gly Asn Arg Asn Ala Phe Lys Gly
   545                 550                 555                 560
   Ala Val Thr Thr Ser Pro Leu Pro Gly Thr Thr Leu Lys Phe Ile Lys
   565 570 575
   Val Asp Leu Gly Gly Gly Arg Ser Leu Tyr Asp Thr Pro Gly Leu Leu
   580 585 590
   Val Leu Gly Thr Val Thr Gln Leu Leu Thr Pro Glu Glu Leu Lys Ile
   595 600 605
   Val Val Pro Lys Lys Pro Ile Glu Pro Val Thr Leu Arg Leu Ser Thr
   610 615 620
   Gly Lys Cys Val Leu Val Gly Gly Leu Ala Arg Ile Glu Leu Ile Gly
   625 630 635 640
   Asp Ser Arg Pro Phe Met Phe Thr Phe Phe Val Ala Asn Glu Ile Lys
   645 650 655
   Leu His Pro Thr Asp Ile Glu Arg Ala Asp Glu Phe Val Leu Lys His
   660 665 670
   Ala Gly Gly Met Leu Thr Pro Pro Leu Ala Pro Gly Pro Lys Arg Met
   675 680 685
   Glu Glu Ile Gly Glu Phe Glu Asp His Ile Val Asp Ile Gln Gly Ala
   690 695 700
   Gly Trp Lys Glu Ala Ala Ala Asp Ile Ser Leu Thr Gly Leu Gly Trp
   705 710 715 720
   Val Ala Val Thr Gly Ala Gly Thr Ala Gln Val Lys Ile Ser Val Pro
   725 730 735
   Lys Gly Ile Gly Val Ser Val Arg Pro Pro Leu Met Pro Phe Asp Ile
   740 745 750
   Trp Lys Val Ala Ser Lys Tyr Thr Gly Ser Arg Ala Val Arg Lys Ser
   755 760 765
   Ser Lys Leu Ala Asn Gly Lys Arg Arg Lys Gly Val Gly Arg Asn
   770 775 780
<210> 3
   <211> 5262
   <212> DNA
   <213> Artificial Sequence
<220>
   <223> Nannochloropsis gaditana
<400> 3
   attttcttgt tatcttatac acacatattc acaggaggat tgtaattagt gaaactggtg 60
   gcaaagggtt tatcaacggt ggctgcgctt cttcctgccc atcgaacagc aacttcagcc 120
   actcaaacca cattactttt cgccctgtga atttttggtc aacttcatgg caccacactt 180
   gagtggccta aattttcatt cattggtcaa gagatcgtcc gctgcagcgc ttcttttctc 240
   gctcttcatc atgaagctac cctgtgtcgg tgctttccaa ggagtagtgc gggtttggag 300
   ttcagcagtg gctccatcgc gagctgccgt actcaccagc tttatgtctc ccaaacgaca 360
   cgtgctaaaa agaatgccaa tttcagcgtt gtgcaggcgg tcaacaatca tggcgtcccg 420
   gaaagcgggg gcgggacagg gcgagcacga ggcagaaggg gagggaatct caccagaatc 480
   gatctcgtcg acaggaagca acgcaggagg caaaggaatc gggcgcggac cccgaaatcg 540
   acggaaaata gccgtaagcg cggaagagga agagttttca gccctgtccg actcgaggac 600
   ctcggtttcc gaagagaagg actcgattcg aaggccgcgg gtcatttctc gccctccctc 660
   ccgccccgtc aagcgaacga tgaccatcaa ccctaattgg cgggcgcatg gaggccctga 720
   gaacagtata aagggaccgg aggaggccgc ttcgtcgtcc tctggtaccg cggggagcgg 780
   aaaggcccgt gtgggaaaga atgggccgcg gggtgcgagt cccctggggg cggaggtacc 840
   ccgctatgtg gaggatgagg atgaggatgg gatcacgttc ccgaaagata tggtaatccg 900
   aggcttggat agccagagct acgaggaagc gcgccgacag gccgtgctga gcgatgacga 960
   gggcgaggag gaggagtggg ccgacgaggg ggtcatggtg gaggaggagg agggggagga 1020
   ctttgacgag gaggaggagg aggaagactt tgacgaggag gaggaggagg aagactttga 1080
   cgagggggac gaagaggagg aggacggcgc tcaccttccc ccagtgcggc ccgtgtcgat 1140
   ggaggagcgc ctccgcctgg cggagtccgg caatatcttc aacccctacg tagcccggat 1200
   gcacacgcgc gcgggcaccg gggaggggcc gtctggggag gcggaagacc ctggcccgat 1260
   ggacgggggc ggtttgcggt tcttggagga ggatgtgagc ccgggggaga aaagggagga 1320
   agcgcgccgg gcccaggcgc cctccctgcc cgtcaagttc cagtacaagg tggtggtggg 1380
   ggccgggacg tgcccgggct gcgggaacgc gtttcagacc aaaaatgaat cctcccccgg 1440
   cttcctcccc cccgacgtct acgagcggct tcaggcccag atgacggcgc tgcgaccagg 1500
   cgctccccga aagccgaggc cggacgcccc ccccctctcg aagtcagcgg caggggcgct 1560
   gcggaagaag acggagacga ggggggaaga gggggatttg ttccaaggcc tgagcgcaga 1620
   ggaggaggtg gaaatgcttt tgtcgggcaa gagccgtgag gagtttgaaa tagagagagc 1680
   ggcggggaga gggagggagg cccagggcgg ggaggtagat cttgacctcg acgaggaggg 1740
   gaaggaagag aaggagggag agggaaggga aggggaagaa gggggggaag gggaggagga 1800
   ggaggaagaa tttcgtgcgg taatttgcca gcggtgccac aagctgaagc actacgggga 1860
   cgtggaggac gccctgcgcc cggggtggag cgcgaacgag ctgttgacgc cggagcgctt 1920
   ccgggagttg gtgagcgtgg tgcgacggaa acgctgcgcc gtggtgtgtc tggtggacat 1980
   cttcgacttc catgggtccc ttctctacaa cctgccccgc atcgtgggct ccaacccggt 2040
   gctggtggcc gtgaacaagg ctgacctcct ccccgcggac ttcagccagg accgagtccg 2100
   gatctgggtc aagcaggaac tggagaaggt ggggatgacg gacgtgagca cgcgcgacat 2160
   ccacctgatc tcctgcaaga cggggaacaa cgtccggccc ttgctgcggt ccatgaagca 2220
   aatggcgcgc cagcgcaggc gggatctgta cgtgatcggc gcggcaaacg tgggcaagtc 2280
   gaccttcatc aaccggctga ttgagctggg tcggagtgga ggggacgcgc agaggaagaa 2340
   gaagaagaag cagggggaac agagcaaagg cgggtctctg gtcacgacga gcgccttacc 2400
   gggcacgacc ttggacttca tcgaggtgga cctgggggac aaggtctccc tctacgacac 2460
   cccgggcctc atcttgccgc accagatcac cacgctgctg aacacggaag agctcaaggc 2520
   agtaattccc cagaagcgca tcaaccacgt gaccctgcgc ctgaaggagg gcaagagcgt 2580
   cctcctgggc gggctagtcc gcttggacat gctggagggt cggcccttcc tcttcacatt 2640
   ctacgtctcg aacgaggtca agctccacca aacagccacg gaccgggccg gggaattttt 2700
   ggacagtcac ctcggtgaac taatctcgcc gcccttcacg caggaacgcc gagcggccat 2760
   ggggccatgg gtacctcgtg acttcgagat cgaagggacg ggctggaaaa cctcagccgt 2820
   tgacatagtc atctctggct tagggtggat atctgtcacc ggcgcattgg actgtaaggt 2880
   ccgagtcatg gcaccggagg cggtgggtgt gcgtttgcgg agtccattga tgccttacga 2940
   gacctgggcg acaacagcca agtggacggg gctcagggcc gtcaagagtg acaagcaaaa 3000
   agggagcagc agataggaag aggaaagttt taccctgtcg tgaaagtgct gatgaagata 3060
   aactatcaag aaaataatca tgtgcattca caaaaacagc tcgttgttgt ttttttgttt 3120
   cctctttgat cagacctttg gtgatattcg attctatttc ttgcccgcat ctcataggat 3180
   tgcgattgta atctccgcaa cagcgttcaa ccagaaaacg tacaacgatc tgagcaaagg 3240
   atagatggct aactctggga ggtcttttgc ttggtatttg tagccggaag atggctttgt 3300
   ggtccagggc ttgtattttc tggcccccac gactgatcta cgtccagagg ggtctgaggg 3360
   actccatgca agccaccaag cacgtacatt tcacacgctc gtacattacg gcattttacg 3420
   ctccgatatc gcgccacggg aatgattggt cacgcccatc aaagcactcg ccggttcttt 3480
   catctctata ctcaaatatc cttacccttc ctcgacagtc gacgcacgat gaacttcgcg 3540
   gacgctgcca cgacgcactc agccgtggaa ggccatgcct cccaggggac cgaaagggag 3600
   gaccaagctg gtggtgacgc tggaaggacc tccctagcca tggcatgacc ggccctcttc 3660
   acgacgtgaa ggacacagaa gacgacaccg actgcgttgt ttatgtcctc aattttcacc 3720
   cccgcaacac ggcggacaag gaaggcaaga tcaacaatgc gatcaacttc gccgatgcag 3780
   aattggagga acatcacgtc ctcctgggag tgtttcttgg tcaatgtgtc gtttcccacc      3840
   tccttccacc caaacgcaat tctaatacct tgtaccttgt tgcccgaata ccaacacctc      3900
   gtacccctaa cacgcacaac acgctttccg acaacgccgg aagactcccc gtccgtacac      3960
   gcgaccccct taaccgcgcc gacatcgagc cggactacta caagcccttc gtctcccctg     4020
   agcttgtcaa caaacgcaat gcagaggcgc acaagcacaa ttcgcctcta ctccgcggtg      4080
   acctaaacgt gacgtacgag cgtacacagg acaagggctt cttggaactt ggccgtgcgc      4140
   ggagtgccac aaaatttgag tcctggcttg gtcttcgcat cataatcgaa ggtccagctt      4200
   tgaacgaccc cggcccaagc aacacgagca gatcgtgtgg gtcgatgaat acaagtttac      4260
   caatgggcag gatttcaagg tgttcttttg gggcgggagc ctgtggcagc tgtgcaagaa      4320
   gtgggccgat gcgaaccgca ctcaatcggg gggagacgac atggtgcgtt tttgtttcga      4380
   ggcccactcc gcttggtgtt cgtgcatgct cacaacgtcc agacgcacct cttctagaca      4440
   caggaccggg cggtggaggc gggctacata gcgccatcct tggatgttgg cgccacgaaa      4500
   gccctggtag catccagtgg aatagaaacc ctcttccagc acgtggctgt gataaagaac      4560
   aagtaagcgc acatccatga ttggacatct tacccttccg tgcatgcatt tctggttgct      4620
   cgttccgctc atttatggac gccatcccta cagagcaaag gatctcgatt gaggaggcgg      4680
   ctttgggaga gcagcagcgg gagatggtcc ggaggaccaa cacaatgggg gaaatcatcg      4740
   gtgtttgtct tgctctccgg accctgtaca acctttaaaa attgccccgt tctcgccttt      4800
   cacggagcaa gatggcgctc ggcgttgcaa gcgcccagca caatgcgctc gcagaccacg      4860
   ccggcataga cctgtcggac gtcgcctcgc ttgacttcca cagcagcagc ggcggccgca      4920
   tgcactcacg gggggatcac atcgaaaccc gcgcagacgc agaggacgcg aaaagcatcc      4980
   tcaaccgtct gaagggtggg aaagaaaaca tccacgagct tcggcagtgt gatttctcac      5040
   ccgtacgccc caagccgggg acgaaagggt actctccaca agaattttta tttcgtcggc      5100
   cgtgaagcgc gtcaagtgct ccacgtgaat gcggaggaag gcgaaaacgg catggagggt      5160
   acgcggcctt gtaggatcgt tgtaccgtgg ctcagggttc gcactcacgc cattgctaag      5220
   gatggttaag aaagttgagc taaaaaaaaa catccctaac ca                         5262
<210> 4
   <211> 949
   <212> PRT
   <213> Artificial Sequence
<220>
   <223> Nannochloropsis gaditana
<400> 4
   Met Ala Pro His Leu Ser Gly Leu Asn Phe His Ser Leu Val Lys Arg
   1               5                   10                  15
   Ser Ser Ala Ala Ala Leu Leu Phe Ser Leu Phe Ile Met Lys Leu Pro
               20                  25                  30
   Cys Val Gly Ala Phe Gln Gly Val Val Arg Val Trp Ser Ser Ala Val
           35                  40                  45
   Ala Pro Ser Arg Ala Ala Val Leu Thr Ser Phe Met Ser Pro Lys Arg
       50                  55                  60
   His Val Leu Lys Arg Met Pro Ile Ser Ala Leu Cys Arg Arg Ser Thr
   65                  70                  75                  80
   Ile Met Ala Ser Arg Lys Ala Gly Ala Gly Gln Gly Glu His Glu Ala
                   85                  90                  95
   Glu Gly Glu Gly Ile Ser Pro Glu Ser Ile Ser Ser Thr Gly Ser Asn
               100                 105                 110
   Ala Gly Gly Lys Gly Ile Gly Arg Gly Pro Arg Asn Arg Arg Lys Ile
           115                 120                 125
   Ala Val Ser Ala Glu Glu Glu Glu Phe Ser Ala Leu Ser Asp Ser Arg
       130                 135                 140
   Thr Ser Val Ser Glu Glu Lys Asp Ser Ile Arg Arg Pro Arg Val Ile
   145                 150                 155                 160
   Ser Arg Pro Pro Ser Arg Pro Val Lys Arg Thr Met Thr Ile Asn Pro
                   165                 170                 175
   Asn Trp Arg Ala His Gly Gly Pro Glu Asn Ser Ile Lys Gly Pro Glu
               180                 185                 190
   Glu Ala Ala Ser Ser Ser Ser Gly Thr Ala Gly Ser Gly Lys Ala Arg
           195                 200                 205
   Val Gly Lys Asn Gly Pro Arg Gly Ala Ser Pro Leu Gly Ala Glu Val
       210                 215                 220
   Pro Arg Tyr Val Glu Asp Glu Asp Glu Asp Gly Ile Thr Phe Pro Lys
   225                 230                 235                 240
   Asp Met Val Ile Arg Gly Leu Asp Ser Gln Ser Tyr Glu Glu Ala Arg
                   245                 250                 255
   Arg Gln Ala Val Leu Ser Asp Asp Glu Gly Glu Glu Glu Glu Trp Ala
               260                 265                 270
   Asp Glu Gly Val Met Val Glu Glu Glu Glu Gly Glu Asp Phe Asp Glu
           275                 280                 285
   Glu Glu Glu Glu Glu Asp Phe Asp Glu Glu Glu Glu Glu Glu Asp Phe
       290                 295                 300
   Asp Glu Gly Asp Glu Glu Glu Glu Asp Gly Ala His Leu Pro Pro Val
   305                 310                 315                 320
   Arg Pro Val Ser Met Glu Glu Arg Leu Arg Leu Ala Glu Ser Gly Asn
                   325                 330                 335
   Ile Phe Asn Pro Tyr Val Ala Arg Met His Thr Arg Ala Gly Thr Gly
               340                 345                 350
   Glu Gly Pro Ser Gly Glu Ala Glu Asp Pro Gly Pro Met Asp Gly Gly
           355                 360                 365
   Gly Leu Arg Phe Leu Glu Glu Asp Val Ser Pro Gly Glu Lys Arg Glu
       370                 375                 380
   Glu Ala Arg Arg Ala Gln Ala Pro Ser Leu Pro Val Lys Phe Gln Tyr
   385                 390                 395                 400
   Lys Val Val Val Gly Ala Gly Thr Cys Pro Gly Cys Gly Asn Ala Phe
                   405                 410                 415
   Gln Thr Lys Asn Glu Ser Ser Pro Gly Phe Leu Pro Pro Asp Val Tyr
               420                 425                 430
   Glu Arg Leu Gln Ala Gln Met Thr Ala Leu Arg Pro Gly Ala Pro Arg
           435                 440                 445
   Lys Pro Arg Pro Asp Ala Pro Pro Leu Ser Lys Ser Ala Ala Gly Ala
       450                 455                 460
   Leu Arg Lys Lys Thr Glu Thr Arg Gly Glu Glu Gly Asp Leu Phe Gln
   465                 470                 475                 480
   Gly Leu Ser Ala Glu Glu Glu Val Glu Met Leu Leu Ser Gly Lys Ser
                   485                 490                 495
   Arg Glu Glu Phe Glu Ile Glu Arg Ala Ala Gly Arg Gly Arg Glu Ala
               500                 505                 510
   Gln Gly Gly Glu Val Asp Leu Asp Leu Asp Glu Glu Gly Lys Glu Glu
   515 520 525
   Lys Glu Gly Glu Gly Arg Glu Gly Glu Glu Gly Gly Glu Gly Glu Glu
   530 535 540
   Glu Glu Glu Glu Phe Arg Ala Val Ile Cys Gln Arg Cys His Lys Leu
   545 550 555 560
   Lys His Tyr Gly Asp Val Glu Asp Ala Leu Arg Pro Gly Trp Ser Ala
   565 570 575
   Asn Glu Leu Leu Thr Pro Glu Arg Phe Arg Glu Leu Val Ser Val Val
   580 585 590
   Arg Arg Lys Arg Cys Ala Val Val Cys Leu Val Asp Ile Phe Asp Phe
   595 600 605
   His Gly Ser Leu Leu Tyr Asn Leu Pro Arg Ile Val Gly Ser Asn Pro
   610 615 620
   Val Leu Val Ala Val Asn Lys Ala Asp Leu Leu Pro Ala Asp Phe Ser
   625 630 635 640
   Gln Asp Arg Val Arg Ile Trp Val Lys Gln Glu Leu Glu Lys Val Gly
   645 650 655
   Met Thr Asp Val Ser Thr Arg Asp Ile His Leu Ile Ser Cys Lys Thr
   660 665 670
   Gly Asn Asn Val Arg Pro Leu Leu Arg Ser Met Lys Gln Met Ala Arg
   675 680 685
   Gln Arg Arg Arg Asp Leu Tyr Val Ile Gly Ala Ala Asn Val Gly Lys
   690 695 700
   Ser Thr Phe Ile Asn Arg Leu Ile Glu Leu Gly Arg Ser Gly Gly Asp
   705 710 715 720
   Ala Gln Arg Lys Lys Lys Lys Lys Gln Gly Glu Gln Ser Lys Gly Gly
   725 730 735
   Ser Leu Val Thr Thr Ser Ala Leu Pro Gly Thr Thr Leu Asp Phe Ile
   740 745 750
   Glu Val Asp Leu Gly Asp Lys Val Ser Leu Tyr Asp Thr Pro Gly Leu
   755 760 765
   Ile Leu Pro His Gln Ile Thr Thr Leu Leu Asn Thr Glu Glu Leu Lys
       770                 775                 780
   Ala Val Ile Pro Gln Lys Arg Ile Asn His Val Thr Leu Arg Leu Lys
   785                 790                 795                 800
   Glu Gly Lys Ser Val Leu Leu Gly Gly Leu Val Arg Leu Asp Met Leu
                   805                 810                 815
   Glu Gly Arg Pro Phe Leu Phe Thr Phe Tyr Val Ser Asn Glu Val Lys
               820                 825                 830
   Leu His Gln Thr Ala Thr Asp Arg Ala Gly Glu Phe Leu Asp Ser His
           835                 840                 845
   Leu Gly Glu Leu Ile Ser Pro Pro Phe Thr Gln Glu Arg Arg Ala Ala
       850                 855                 860
   Met Gly Pro Trp Val Pro Arg Asp Phe Glu Ile Glu Gly Thr Gly Trp
   865                 870                 875                 880
   Lys Thr Ser Ala Val Asp Ile Val Ile Ser Gly Leu Gly Trp Ile Ser
                   885                 890                 895
   Val Thr Gly Ala Leu Asp Cys Lys Val Arg Val Met Ala Pro Glu Ala
               900                 905                 910
   Val Gly Val Arg Leu Arg Ser Pro Leu Met Pro Tyr Glu Thr Trp Ala
           915                 920                 925
   Thr Thr Ala Lys Trp Thr Gly Leu Arg Ala Val Lys Ser Asp Lys Gln
       930                 935                 940
   Lys Gly Ser Ser Arg
   945
<210> 5
   <211> 2874
   <212> DNA
   <213> Artificial Sequence
<220>
   <223> Codon-optimized NgNOA-encoding sequence
<400> 5
   ggatcctcta gaatggctcc ccacctctcc ggcctcaact tccactccct cgtcaagcgc      60
   tcctccgctg ctgctctcct cttctccctc ttcatcatga agctcccctg cgtcggcgct     120
   ttccagggcg tcgtccgcgt ctggtcctcc gctgtcgctc cctcccgcgc tgctgtcctc     180
   acctccttca tgtcccccaa gcgccacgtc ctcaagcgca tgcccatctc cgctctctgc     240
   cgccgctcca ccatcatggc ttcccgcaag gctggcgctg gccagggcga gcacgaggct     300
   gagggcgagg gcatctcccc cgagtccatc tcctccaccg gctccaacgc tggcggcaag     360
   ggcatcggcc gcggcccccg caaccgccgc aagatcgctg tctccgctga ggaggaggag     420
   ttctccgctc tctccgactc ccgcacctcc gtctccgagg agaaggactc catccgccgc     480
   ccccgcgtca tctcccgccc cccctcccgc cccgtcaagc gcaccatgac catcaacccc     540
   aactggcgcg ctcacggcgg ccccgagaac tccatcaagg gccccgagga ggctgcttcc     600
   tcctcctccg gcaccgctgg ctccggcaag gctcgcgtcg gcaagaacgg cccccgcggc     660
   gcttcccccc tcggcgctga ggtcccccgc tacgtcgagg acgaggacga ggacggcatc     720
   accttcccca aggacatggt catccgcggc ctcgactccc agtcctacga ggaggctcgc     780
   cgccaggctg tcctctccga cgacgagggc gaggaggagg agtgggctga cgagggcgtc     840
   atggtcgagg aggaggaggg cgaggacttc gacgaggagg aggaggagga ggacttcgac     900
   gaggaggagg aggaggagga cttcgacgag ggcgacgagg aggaggagga cggcgctcac     960
   ctcccccccg tccgccccgt ctccatggag gagcgcctcc gcctcgctga gtccggcaac    1020
   atcttcaacc cctacgtcgc tcgcatgcac acccgcgctg gcaccggcga gggcccctcc    1080
   ggcgaggctg aggaccccgg ccccatggac ggcggcggcc tccgcttcct cgaggaggac    1140
   gtctcccccg gcgagaagcg cgaggaggct cgccgcgctc aggctccctc cctccccgtc    1200
   aagttccagt acaaggtcgt cgtcggcgct ggcacctgcc ccggctgcgg caacgctttc    1260
   cagaccaaga acgagtcctc ccccggcttc ctcccccccg acgtctacga gcgcctccag    1320
   gctcagatga ccgctctccg ccccggcgct ccccgcaagc cccgccccga cgctcccccc    1380
   ctctccaagt ccgctgctgg cgctctccgc aagaagaccg agacccgcgg cgaggagggc    1440
   gacctcttcc agggcctctc cgctgaggag gaggtcgaga tgctcctctc cggcaagtcc    1500
   cgcgaggagt tcgagatcga gcgcgctgct ggccgcggcc gcgaggctca gggcggcgag    1560
   gtcgacctcg acctcgacga ggagggcaag gaggagaagg agggcgaggg ccgcgagggc    1620
   gaggagggcg gcgagggcga ggaggaggag gaggagttcc gcgctgtcat ctgccagcgc    1680
   tgccacaagc tcaagcacta cggcgacgtc gaggacgctc tccgccccgg ctggtccgct    1740
   aacgagctcc tcacccccga gcgcttccgc gagctcgtct ccgtcgtccg ccgcaagcgc    1800
   tgcgctgtcg tctgcctcgt cgacatcttc gacttccacg gctccctcct ctacaacctc    1860
   ccccgcatcg tcggctccaa ccccgtcctc gtcgctgtca acaaggctga cctcctcccc    1920
   gctgacttct cccaggaccg cgtccgcatc tgggtcaagc aggagctcga gaaggtcggc    1980
   atgaccgacg tctccacccg cgacatccac ctcatctcct gcaagaccgg caacaacgtc    2040
   cgccccctcc tccgctccat gaagcagatg gctcgccagc gccgccgcga cctctacgtc 2100
   atcggcgctg ctaacgtcgg caagtccacc ttcatcaacc gcctcatcga gctcggccgc 2160
   tccggcggcg acgctcagcg caagaagaag aagaagcagg gcgagcagtc caagggcggc 2220
   tccctcgtca ccacctccgc tctccccggc accaccctcg acttcatcga ggtcgacctc 2280
   ggcgacaagg tctccctcta cgacaccccc ggcctcatcc tcccccacca gatcaccacc 2340
   ctcctcaaca ccgaggagct caaggctgtc atcccccaga agcgcatcaa ccacgtcacc 2400
   ctccgcctca aggagggcaa gtccgtcctc ctcggcggcc tcgtccgcct cgacatgctc 2460
   gagggccgcc ccttcctctt caccttctac gtctccaacg aggtcaagct ccaccagacc 2520
   gctaccgacc gcgctggcga gttcctcgac tcccacctcg gcgagctcat ctcccccccc 2580
   ttcacccagg agcgccgcgc tgctatgggc ccctgggtcc cccgcgactt cgagatcgag 2640
   ggcaccggct ggaagacctc cgctgtcgac atcgtcatct ccggcctcgg ctggatctcc 2700
   gtcaccggcg ctctcgactg caaggtccgc gtcatggctc ccgaggctgt cggcgtccgc 2760
   ctccgctccc ccctcatgcc ctacgagacc tgggctacca ccgctaagtg gaccggcctc 2820
   cgcgctgtca agtccgacaa gcagaagggc tcctcccgct aagaattcca tatg 2874
<210> 6
   <211> 6605
   <212> DNA
   <213> Artificial Sequence
<220>
   <223> pH4-NOAOE
<400> 6
   ctaaattgta agcgttaata ttttgttaaa attcgcgtta aatttttgtt aaatcagctc      60
   attttttaac caataggccg aaatcggcaa aatcccttat aaatcaaaag aatagaccga      120
   gatagggttg agtgttgttc cagtttggaa caagagtcca ctattaaaga acgtggactc      180
   caacgtcaaa gggcgaaaaa ccgtctatca gggcgatggc ccactacgtg aaccatcacc      240
   ctaatcaagt tttttggggt cgaggtgccg taaagcacta aatcggaacc ctaaagggag      300
   cccccgattt agagcttgac ggggaaagcc ggcgaacgtg gcgagaaagg aagggaagaa      360
   agcgaaagga gcgggcgcta gggcgctggc aagtgtagcg gtcacgctgc gcgtaaccac      420
   cacacccgcc gcgcttaatg cgccgctaca gggcgcgtcc cattcgccat tcaggctgcg      480
   caactgttgg gaagggcgat cggtgcgggc ctcttcgcta ttacgccagc tggcgaaagg      540
   gggatgtgct gcaaggcgat taagttgggt aacgccaggg ttttcccagt cacgacgttg      600
   taaaacgacg gccagtgagc gcgcgtaata cgactcacta tagggcgaat tggagctcgc      660
   aatctcacgc accaggcgct ggaagggcaa cttgcggatg agaaggtccg tggacttctg      720
   gtaacgacgg atctcacgca gagcgacggt tccagggcga taacggtggg gcttcttgac 780
   tcctccggta gccggagcgg acttgcgggc agccttggtg gcaagctgct tgcgcggcgc 840
   tttgcctccg gtggatttac gggcggtttg cttggttcgg gccattttga cggttttttt 900
   tacaagagaa gagttcttga aatttgtgag gttaaagtgt gtggcttccg ccgtagtcaa 960
   ggagcgtgcg gttgccgatc gcaccggtac gttctgtaga aatgaacaca gtgtgttgaa 1020
   ttgaaagtat ggcgcaggta tggtgtgtga taagtagcag ccgcgccgag acaaacaaac 1080
   tttggtttct acgacaatct ctgtagacaa gtactagaaa cccgtttgaa cgagcataaa 1140
   tctgcaccgg caggccacca gacatcgttt caacgtaata ttctacgtaa ccattttatc 1200
   ccaggaaacc tacggcctgt gaaccaccga gacggagcac tcacaattcg ctctcggcaa 1260
   caaccgacaa tcgtcttact cacagtcaat accgaaaaca aacaacagcc atggccaagt 1320
   tgaccagtgc cgttccggtg ctcaccgcgc gcgacgtcgc cggagcggtc gagttctgga 1380
   ccgaccggct cgggttctcc cgggacttcg tggaggacga cttcgccggt gtggtccggg 1440
   acgacgtgac cctgttcatc agcgcggtcc aggaccaggt ggtgccggac aacaccctgg 1500
   cctgggtgtg ggtgcgcggc ctggacgagc tgtacgccga gtggtcggag gtcgtgtcca 1560
   cgaacttccg ggacgcctcc gggccggcca tgaccgagat cggcgagcag ccgtgggggc 1620
   gggagttcgc cctgcgcgac ccggccggca actgcgtgca cttcgtggcc gaggagcagg 1680
   actgaccgac gccgaccaac accgccggtc cgacggcggc ccacgggtcc caggccttct 1740
   agaatggtcc ccactggttg tatgagatca aagaacaaac agagtactgc tgctgacgga 1800
   agaaggtcga ttggaagtcg atccaacgct ggactggtca tgttctttag tatgactatc 1860
   ttatcgacac gacgatttgc ggcagcttgg gtttttcaga gtagtggaat acaacgatca 1920
   ataccatcca cgcataggat gcgaacgaat tttgctttgt cgacgcgctg ctttgcttct 1980
   tcatccgaca accatgacga agaggaacaa cgagactctc cgaaacaaag atccaaacgc 2040
   agccaaacta atcggtccaa gaaattcaaa attgctgaat caatcgacca gagcaaaata 2100
   gataagctag cacaagcatt cgatgaactc gctcggaagg aaggcttcga ctcgtcaaca 2160
   gcacgctttg ccgacgatgt gacgttcgag gacaagtttg acgacgattc gtttctggac 2220
   gatgacgatg ataacaacaa agataaagtg ggaaacttgc acctagatgc atccatgttc 2280
   agtttaagtg actttataga taagagtgag gaagatggcg gcaatccaac cgatcaagat 2340
   gacgaggact accttgattt tggtgcagac attgacatga gtatagaagc aaggattgcc 2400
   gctgccaaac gggatatgga tctcggtcga gtcagcgccc ctcccgatat gagatcctcg 2460
   cgcagggagg taactgcagc cgaccttcgc aaacttggat ttcgaaccga ggcaaaccca 2520
   ttcggcaacg acgaaactcc acggaaggag cgcttccagt tggtaacaaa ctccatgtcg 2580
   tgctccgcct gtggatcgga ctttcaatgc cacaacgaag atcggcccgg atatctgcct 2640
   cctgaaaagt tcgctacgca aacagcactt ggaaaaatag aacagatgca aaagttgcag 2700
   gataaagcag aaaaagcgga atggacacct gaagatgaga ttgaatggtt gattcagact 2760
   cagggcaaaa aggatccgaa caaagaaatg caggaggtgc cccagatcga tgttgattct 2820
   ttggcagggg aaatgggcct tgacctcgta gagctttcca aaaagatggt tatttgcaag 2880
   cgctgtcacg gtctgcaaaa ctttggaaaa gtgcaagatt ccctccgacc tgggtggacg 2940
   aaggagccac tgttgtcgca ggagaaattt cgtgaattgt taaggccaat caaggaaaag 3000
   ccggcagtta tcgttgcatt ggtcgatctt tttgattttt cggggtctgt gctccctgag 3060
   cttgatgaaa tcgctggtga aaaccctgta attcttgcgg ccaacaaggc ggatcttctt 3120
   ccaagtgaaa tgggacgcgt gcgagctgag agttgggttc gacgcgagct cgaatacctt 3180
   ggagtcaagt cgttggccgg tatgagagga gcagttcggc ttgtcagctg caagactgga 3240
   gctgggatta atgatttgct ggagaaagca agaggattag ccgaggaaat cgacggcgac 3300
   atatacgtcg tcggggctgc aaatgcagga aaaagtacgc ttttgaattt tgttctaggt 3360
   caggacaagg tgaacagatc acccggaaaa gcacgagcag gcaacaggaa tgccttcaag 3420
   ggcgcggtga cgacaagtcc actgccaggc acaacgctta agttcatcaa agtcgattta 3480
   ggcggcggtc gaagtctata tgacactcct ggtcttctgg tattaggcac tgtgacacag 3540
   ttactgaccc ccgaagagct gaagatagtt gttcccaaaa agtatgtcaa accgatcaaa 3600
   ctgatattcg attcacagtc aataatgttc aaactaacac ctcgttcctc aaacaggcca 3660
   attgaacctg tcaccctccg gctctctacc ggaaagtgcg ttctagttgg aggattggcc 3720
   cgcatcgagt taatcggcga ctcaagaccc tttatgttca cattttttgt tgctaatgag 3780
   atcaagctcc accctactga catagagaga gccgatgagt tcgttctaaa gcacgctggt 3840
   ggcatgttga ctccaccgct agcacccgga ccaaaacgta tggaagagat tggagaattt 3900
   gaagatcaca tcgtggatat ccagggtgct ggctggaaag aagctgctgc tgatatcagt 3960
   cttaccggac taggatgggt ggccgttaca ggagcaggga cagcgcaagt aaaaataagt 4020
   gttccgaaag gtattggtgt atcggtgcgg cctccgctta tgcctttcga tatctggaaa 4080
   gttgcatcga agtataccgg aagtcgagct gtaagaaagt catccaaact ggcgaatggg 4140
   aaacgaagaa aaggtgtagg gcgtaattag gaattctcga gctacctcga ctttggctgg 4200
   gacactttca gtgaggacaa gaagcttcag aagcgtgcta tcgaactcaa ccagggacgt 4260
   gcggcacaaa tgggcatcct tgctctcatg gtgcacgaac agttgggagt ctctatcctt 4320
   ccttaaaaat ttaattttca ttagttgcag tcactccgct ttggtttcac agtcaggaat 4380
   aacactagct cgtcttcagg tacccagctt ttgttccctt tagtgagggt taattgcgcg 4440
   cttggcgtaa tcatggtcat agctgtttcc tgtgtgaaat tgttatccgc tcacaattcc 4500
   acacaacata cgagccggaa gcataaagtg taaagcctgg ggtgcctaat gagtgagcta 4560
   actcacatta attgcgttgc gctcactgcc cgctttccag tcgggaaacc tgtcgtgcca 4620
   gctgcattaa tgaatcggcc aacgcgcggg gagaggcggt ttgcgtattg ggcgctcttc 4680
   cgcttcctcg ctcactgact cgctgcgctc ggtcgttcgg ctgcggcgag cggtatcagc 4740
   tcactcaaag gcggtaatac ggttatccac agaatcaggg gataacgcag gaaagaacat 4800
   gtgagcaaaa ggccagcaaa aggccaggaa ccgtaaaaag gccgcgttgc tggcgttttt 4860
   ccataggctc cgcccccctg acgagcatca caaaaatcga cgctcaagtc agaggtggcg 4920
   aaacccgaca ggactataaa gataccaggc gtttccccct ggaagctccc tcgtgcgctc 4980
   tcctgttccg accctgccgc ttaccggata cctgtccgcc tttctccctt cgggaagcgt 5040
   ggcgctttct catagctcac gctgtaggta tctcagttcg gtgtaggtcg ttcgctccaa 5100
   gctgggctgt gtgcacgaac cccccgttca gcccgaccgc tgcgccttat ccggtaacta 5160
   tcgtcttgag tccaacccgg taagacacga cttatcgcca ctggcagcag ccactggtaa 5220
   caggattagc agagcgaggt atgtaggcgg tgctacagag ttcttgaagt ggtggcctaa 5280
   ctacggctac actagaagga cagtatttgg tatctgcgct ctgctgaagc cagttacctt 5340
   cggaaaaaga gttggtagct cttgatccgg caaacaaacc accgctggta gcggtggttt 5400
   ttttgtttgc aagcagcaga ttacgcgcag aaaaaaagga tctcaagaag atcctttgat 5460
   cttttctacg gggtctgacg ctcagtggaa cgaaaactca cgttaaggga ttttggtcat 5520
   gagattatca aaaaggatct tcacctagat ccttttaaat taaaaatgaa gttttaaatc 5580
   aatctaaagt atatatgagt aaacttggtc tgacagttac caatgcttaa tcagtgaggc 5640
   acctatctca gcgatctgtc tatttcgttc atccatagtt gcctgactcc ccgtcgtgta 5700
   gataactacg atacgggagg gcttaccatc tggccccagt gctgcaatga taccgcgaga 5760
   cccacgctca ccggctccag atttatcagc aataaaccag ccagccggaa gggccgagcg 5820
   cagaagtggt cctgcaactt tatccgcctc catccagtct attaattgtt gccgggaagc 5880
   tagagtaagt agttcgccag ttaatagttt gcgcaacgtt gttgccattg ctacaggcat 5940
   cgtggtgtca cgctcgtcgt ttggtatggc ttcattcagc tccggttccc aacgatcaag 6000
   gcgagttaca tgatccccca tgttgtgcaa aaaagcggtt agctccttcg gtcctccgat 6060
   cgttgtcaga agtaagttgg ccgcagtgtt atcactcatg gttatggcag cactgcataa 6120
   ttctcttact gtcatgccat ccgtaagatg cttttctgtg actggtgagt actcaaccaa 6180
   gtcattctga gaatagtgta tgcggcgacc gagttgctct tgcccggcgt caatacggga 6240
   taataccgcg ccacatagca gaactttaaa agtgctcatc attggaaaac gttcttcggg 6300
   gcgaaaactc tcaaggatct taccgctgtt gagatccagt tcgatgtaac ccactcgtgc 6360
   acccaactga tcttcagcat cttttacttt caccagcgtt tctgggtgag caaaaacagg 6420
   aaggcaaaat gccgcaaaaa agggaataag ggcgacacgg aaatgttgaa tactcatact 6480
   cttccttttt caatattatt gaagcattta tcagggttat tgtctcatga gcggatacat 6540
   atttgaatgt atttagaaaa ataaacaaat aggggttccg cgcacatttc cccgaaaagt 6600
   gccac 6605
<210> 7
   <211> 5198
   <212> DNA
   <213> Artificial Sequence
<220>
   <223> PCT2Ng
<400> 7
   gtggcacttt tcggggaaat gtgcgcggaa cccctatttg tttatttttc taaatacatt      60
   caaatatgta tccgctcatg agacaataac cctgataaat gcttcaataa tattgaaaaa     120
   ggaagagtat gagtattcaa catttccgtg tcgcccttat tccctttttt gcggcatttt     180
   gccttcctgt ttttgctcac ccagaaacgc tggtgaaagt aaaagatgct gaagatcagt     240
   tgggtgcacg agtgggttac atcgaactgg atctcaacag cggtaagatc cttgagagtt     300
   ttcgccccga agaacgtttt ccaatgatga gcacttttaa agttctgcta tgtggcgcgg     360
   tattatcccg tattgacgcc gggcaagagc aactcggtcg ccgcatacac tattctcaga     420
   atgacttggt tgagtactca ccagtcacag aaaagcatct tacggatggc atgacagtaa     480
   gagaattatg cagtgctgcc ataaccatga gtgataacac tgcggccaac ttacttctga     540
   caacgatcgg aggaccgaag gagctaaccg cttttttgca caacatgggg gatcatgtaa     600
   ctcgccttga tcgttgggaa ccggagctga atgaagccat accaaacgac gagcgtgaca     660
   ccacgatgcc tgtagcaatg gcaacaacgt tgcgcaaact attaactggc gaactactta     720
   ctctagcttc ccggcaacaa ttaatagact ggatggaggc ggataaagtt gcaggaccac     780
   ttctgcgctc ggcccttccg gctggctggt ttattgctga taaatctgga gccggtgagc     840
   gtgggtctcg cggtatcatt gcagcactgg ggccagatgg taagccctcc cgtatcgtag     900
   ttatctacac gacggggagt caggcaacta tggatgaacg aaatagacag atcgctgaga     960
   taggtgcctc actgattaag cattggtaac tgtcagacca agtttactca tatatacttt    1020
   agattgattt aaaacttcat ttttaattta aaaggatcta ggtgaagatc ctttttgata    1080
   atctcatgac caaaatccct taacgtgagt tttcgttcca ctgagcgtca gaccccgtag    1140
   aaaagatcaa aggatcttct tgagatcctt tttttctgcg cgtaatctgc tgcttgcaaa    1200
   caaaaaaacc accgctacca gcggtggttt gtttgccgga tcaagagcta ccaactcttt    1260
   ttccgaaggt aactggcttc agcagagcgc agataccaaa tactgtcctt ctagtgtagc    1320
   cgtagttagg ccaccacttc aagaactctg tagcaccgcc tacatacctc gctctgctaa 1380
   tcctgttacc agtggctgct gccagtggcg ataagtcgtg tcttaccggg ttggactcaa 1440
   gacgatagtt accggataag gcgcagcggt cgggctgaac ggggggttcg tgcacacagc 1500
   ccagcttgga gcgaacgacc tacaccgaac tgagatacct acagcgtgag ctatgagaaa 1560
   gcgccacgct tcccgaaggg agaaaggcgg acaggtatcc ggtaagcggc agggtcggaa 1620
   caggagagcg cacgagggag cttccagggg gaaacgcctg gtatctttat agtcctgtcg 1680
   ggtttcgcca cctctgactt gagcgtcgat ttttgtgatg ctcgtcaggg gggcggagcc 1740
   tatggaaaaa cgccagcaac gcggcctttt tacggttcct ggccttttgc tggccttttg 1800
   ctcacatgtt ctttcctgcg ttatcccctg attctgtgga taaccgtatt accgcctttg 1860
   agtgagctga taccgctcgc cgcagccgaa cgaccgagcg cagcgagtca gtgagcgagg 1920
   aagcggaaga gcgcccaata cgcaaaccgc ctctccccgc gcgttggccg attcattaat 1980
   gcagctggca cgacaggttt cccgactgga aagcgggcag tgagcgcaac gcaattaatg 2040
   tgagttagct cactcattag gcaccccagg ctttacactt tatgcttccg gctcgtatgt 2100
   tgtgtggaat tgtgagcgga taacaatttc acacaggaaa cagctatgac catgattacg 2160
   ccaagcgcgc aattaaccct cactaaaggg aacaaaagct ggagctcagc tgctgccccg 2220
   accgtatctc caagtcagac atgaaatctt cagttgcgtt aaaaactcta cgatgctacc 2280
   agcgttaaat aaccttgccc acgcctttaa acgtacccga tcattaacat atcgactggc 2340
   tgccttggct ttgcaccagc catcatcaga cttaacgatg ggtatgttgc ttgcctttcc 2400
   tgcttgaagg gggtccgact ctctgctttc tcgatcgcgg gtgtgacctc tgaattggaa 2460
   tgtaaaaatg taagaagcga cgtgtccggt aaagaaatgc ccaagctcca tcaaatctgc 2520
   gttgtcggcg accaaaccat gctggctcgt cgacctgccc cggatgcagg agcatggcac 2580
   tcggcggcat ggcacttgag cctcgcggga ggaatgtgtg tggttgggcg caggctgtgg 2640
   acggcccccc tccagcgaag cggtcgcctc cctttccgac gctttgtgca cgttgtctgg 2700
   tgtcctctgt ctcacgcacc tcttcaccga cgtggtgtcc ctcttgttgc tggtgaggga 2760
   cttggaatgt ggtcctggtt ctatcctggg cgcgtgtgtt cctttttttc tctaccgtta 2820
   ttctctccat ttctgatgtc tcaccaccat ctccctcacc ctccaaccgc gtcgttgtgc 2880
   caaaatcata cagcaggatc gatggccaag ttgaccagtg ccgttccggt gctcaccgcg 2940
   cgcgacgtcg ccggagcggt cgagttctgg accgaccggc tcgggttctc ccgggacttc 3000
   gtggaggacg acttcgccgg tgtggtccgg gacgacgtga ccctgttcat cagcgcggtc 3060
   caggaccagg tggtgccgga caacaccctg gcctgggtgt gggtgcgcgg cctggacgag 3120
   ctgtacgccg agtggtcgga ggtcgtgtcc acgaacttcc gggacgcctc cgggccggcc 3180
   atgaccgaga tcggcgagca gccgtggggg cgggagttcg ccctgcgcga cccggccggc 3240
   aactgcgtgc acttcgtggc cgaggagcag gactaaatcg atcttcctta aaaatttaat 3300
   tttcattagt tgcagtcact ccgctttggt ttcacagtca ggaataacac tagctcgtct 3360
   tcaccatgga tgccaatctc gcctattcat ggtgtataaa agttcaacat ccaaagctag 3420
   aacttttgga aagagaaaga atatccgaat agggcacggc gtgccgtatt gttggagtgg 3480
   actagcagaa agtgaggaag gcacaggatg agttttctcg agagctgctg ccccgaccgt 3540
   atctccaagt cagacatgaa atcttcagtt gcgttaaaaa ctctacgatg ctaccagcgt 3600
   taaataacct tgcccacgcc tttaaacgta cccgatcatt aacatatcga ctggctgcct 3660
   tggctttgca ccagccatca tcagacttaa cgatgggtat gttgcttgcc tttcctgctt 3720
   gaagggggtc cgactctctg ctttctcgat cgcgggtgtg acctctgaat tggaatgtaa 3780
   aaatgtaaga agcgacgtgt ccggtaaaga aatgcccaag ctccatcaaa tctgcgttgt 3840
   cggcgaccaa accatgctgg ctcgtcgacc tgccccggat gcaggagcat ggcactcggc 3900
   ggcatggcac ttgagcctcg cgggaggaat gtgtgtggtt gggcgcaggc tgtggacggc 3960
   ccccctccag cgaagcggtc gcctcccttt ccgacgcttt gtgcacgttg tctggtgtcc 4020
   tctgtctcac gcacctcttc accgacgtgg tgtccctctt gttgctggtg agggacttgg 4080
   aatgtggtcc tggttctatc ctgggcgcgt gtgttccttt ttttctctac cgttattctc 4140
   tccatttctg atgtctcacc accatctccc tcaccctcca accgcgtcgt tgtgccaaaa 4200
   tcatacagca ggaggcctgt cgacggcgcg ccggatccag atctgaattc gatatcacgc 4260
   gtccatggca tatggctagc gcggccgcct cgagtctaga cttccttaaa aatttaattt 4320
   tcattagttg cagtcactcc gctttggttt cacagtcagg aataacacta gctcgtcttc 4380
   accatggatg ccaatctcgc ctattcatgg tgtataaaag ttcaacatcc aaagctagaa 4440
   cttttggaaa gagaaagaat atccgaatag ggcacggcgt gccgtattgt tggagtggac 4500
   tagcagaaag tgaggaaggc acaggatgag ttttctcgag ggtacccaat tcgccctata 4560
   gtgagtcgta ttacgcgcgc tcactggccg tcgttttaca acgtcgtgac tgggaaaacc 4620
   ctggcgttac ccaacttaat cgccttgcag cacatccccc tttcgccagc tggcgtaata 4680
   gcgaagaggc ccgcaccgat cgcccttccc aacagttgcg cagcctgaat ggcgaatggg 4740
   acgcgccctg tagcggcgca ttaagcgcgg cgggtgtggt ggttacgcgc agcgtgaccg 4800
   ctacacttgc cagcgcccta gcgcccgctc ctttcgcttt cttcccttcc tttctcgcca 4860
   cgttcgccgg ctttccccgt caagctctaa atcgggggct ccctttaggg ttccgattta 4920
   gtgctttacg gcacctcgac cccaaaaaac ttgattaggg tgatggttca cgtagtgggc 4980
   catcgccctg atagacggtt tttcgccctt tgacgttgga gtccacgttc tttaatagtg 5040
   gactcttgtt ccaaactgga acaacactca accctatctc ggtctattct tttgatttat 5100
   aagggatttt gccgatttcg gcctattggt taaaaaatga gctgatttaa caaaaattta 5160
   acgcgaattt taacaaaata ttaacgctta caatttag 5198
<210> 8
   <211> 31
   <212> DNA
   <213> Artificial Sequence
<220>
   <223> primer
<400> 8
   tttatctaga atggtcccca ctggttgtat g 31
<210> 9
   <211> 36
   <212> DNA
   <213> Artificial Sequence
<220>
   <223> primer
<400> 9
   tttagaattc ctaattacgc cctacacctt ttcttc 36
<210> 10
   <211> 21
   <212> DNA
   <213> Artificial Sequence
<220>
   <223> primer
<400> 10
   cgaagtcaac caggaaacca a 21
<210> 11
   <211> 21
   <212> DNA
   <213> Artificial Sequence
<220>
   <223> primer
<400> 11
   gtgcaagaga ccggacatac c 21
<210> 12
   <211> 21
   <212> DNA
   <213> Artificial Sequence
<220>
   <223> primer
<400> 12
   ctgggagctt tactgcttgg a 21
   <210> 13
   <211> 21
   <212> DNA
   <213> Artificial Sequence
<220>
   <223> primer
<400> 13
   atggctcgag atcgacgtaa a 21
<210> 14
   <211> 20
   <212> DNA
   <213> Artificial Sequence
<220>
   <223> primer
<400> 14
   cctgaaaagt tcgctacgca 20
<210> 15
   <211> 20
   <212> DNA
   <213> Artificial Sequence
<220>
   <223> primer
<400> 15
   cggatccttt ttgccctgag 20

## Claims

1. A method for increasing the production of triacylglycerol (TAG) in a microalga, said method comprising:
culturing a recombinant microalga which has been transformed with a recombinant nucleic acid encoding a NOA protein under conditions suitable for the production or overproduction of NO by said microalga, so as to enhance the production of said triacylglycerol,
wherein the triacylglycerol content is increased in said recombinant microalga to at least 150%, preferably at least 200%, compared to a corresponding microalga wherein the NO production pathway was not modulated.

2. The method according to claim 1, wherein said NOA protein is the *Phaeodactylum tricornutum* NOA (*Pt*NOA) protein having an amino acid sequence of SEQ ID NO:2 or a functional variant thereof that has an amino acid sequence of at least 80% sequence identity with SEQ ID NO:2.

3. The method according to claim 1, wherein said NOA protein is the *Nannochloropsis gaditana* NOA (NgNOA) protein having the amino acid sequence of SEQ ID NO:4 or a functional variant thereof that has an amino acid sequence of at least 80% sequence identity with SEQ ID NO:4.

4. The method according to any one of claims 1 to 3, wherein the microalga is selected from the Chromalveolata.

5. The method according to claim 4, wherein the microalga is selected from the Bacillariophyceae or the Eustigmatophyceae.

6. The method according to claim 5, wherein the microalga is *Phaeodactylum tricornutum.*

7. The method according to any one of claims 1 to 6, wherein the production of triacylglycerol occurs during the growth phase of the recombinant microalga.

8. Use of a recombinant microalga which has been transformed with a recombinant nucleic acid encoding a NOA protein for the production of TAGs wherein the triacylglycerol content is increased in said recombinant microalga to at least 150%, preferably at least 200%, compared to a corresponding microalga wherein the NO production pathway was not modulated.

9. Use according to claim 8 for the production of fatty acids, hydrocarbons or fatty alcohols.

10. Use according to any one of claims 8 or 9 for biofuel production or for the production of cosmetics.

11. A recombinant microalga, which has been transformed with a recombinant nucleic acid encoding a *Pt*NOA homolog that is from *Nannochloropsis gaditana* and that has the amino acid sequence of SEQ ID NO:4, wherein the triacylglycerol content is increased in said recombinant microalga to at least 150%, preferably at least 200%, compared to a corresponding microalga wherein the NO production pathway was not modulated.

## Patentansprüche

1. Verfahren zum Erhöhen der Herstellung von Triacylglycerol (TAG) in einer Mikroalge, wobei das Verfahren umfasst:
Kultivieren einer rekombinanten Mikroalge, die mit einer rekombinanten, für ein NOA-Protein kodierenden Nukleinsäure transformiert wurde, unter Bedingungen, die für die Herstellung oder Überproduktion von NO durch die Mikroalge geeignet sind, um die Herstellung des Triacylglycerols zu erhöhen,
wobei der Triacylglycerolgehalt in der rekombinanten Mikroalge auf mindestens 150 %, vorzugsweise mindestens 200 %, verglichen mit einer entsprechenden Mikroalge, bei der der NO-Produktionsweg nicht moduliert wurde, erhöht ist.

2. Verfahren nach Anspruch 1, wobei das NOA-Protein das *Phaeodactylum-tricornutum-*NOA-Protein (*Pt*-NOA-Protein) mit einer Aminosäuresequenz von SEQ ID NO: 2 oder eine funktionelle Variante davon ist, die eine Aminosäuresequenz von mindestens 80 % Sequenzidentität zu SEQ ID NO: 2 aufweist.

3. Verfahren nach Anspruch 1, wobei das NOA-Protein das *Nannochloropsis gaditana-NOA-*Protein (*Ng*-NOA-Protein) mit einer Aminosäuresequenz von SEQ ID NO: 4 oder eine funktionelle Variante davon ist, die eine Aminosäuresequenz von mindestens 80 % Sequenzidentität zu SEQ ID NO: 4 aufweist.

4. Verfahren nach einem der Ansprüche 1 bis 3, wobei die Mikroalge ausgewählt ist aus den Chromalveolata.

5. Verfahren nach Anspruch 4, wobei die Mikroalge ausgewählt ist aus Bacillariophyceae oder Eustigmatophyceae.

6. Verfahren nach Anspruch 5, wobei die Mikroalge *Phaeodactylum tricornutum* ist.

7. Verfahren nach einem der Ansprüche 1 bis 6, wobei die Herstellung von Triacylglycerol während der Wachstumsphase der rekombinanten Mikroalge erfolgt.

8. Verwendung einer rekombinanten Mikroalge, die mit einer rekombinanten, für ein NOA-Protein kodierenden Nukleinsäure transformiert wurde, zur Herstellung von TAG, wobei der Triacylglycerolgehalt in der rekombinanten Mikroalge auf mindestens 150 %, vorzugsweise mindestens 200 %, verglichen mit einer entsprechenden Mikroalge, bei der der NO-Produktionsweg nicht moduliert wurde, erhöht ist.

9. Verwendung nach Anspruch 8 zur Herstellung von Fettsäuren, Kohlenwasserstoffen oder Fettalkoholen.

10. Verwendung nach einem der Ansprüche 8 oder 9 zur Herstellung von Biokraftstoffen oder zur Herstellung von Kosmetika.

11. Rekombinante Mikroalge, die mit einer rekombinanten Nukleinsäure transformiert wurde, die für ein *Pt*-NOA-Homolog kodiert, das aus *Nannochloropsis gaditana* stammt und die Aminosäuresequenz von SEQ ID NO: 4 aufweist, wobei der Triacylglycerolgehalt in der rekombinanten Mikroalge auf mindestens 150 %, vorzugsweise mindestens 200 %, verglichen mit einer entsprechenden Mikroalge, bei der der NO-Produktionsweg nicht moduliert wurde, erhöht ist.

## Revendications

1. Procédé pour accroître la production de triacylglycérol (TAG) dans une microalgue, ledit procédé comprenant :
la mise en culture d'une microalgue recombinante qui a été transformée avec un acide nucléique recombinant codant pour une protéine NOA dans des conditions appropriées pour la production ou la surproduction de NO par ladite microalgue, de manière à renforcer la production dudit triacylglycérol,
dans lequel la teneur en triacylglycérol est accrue dans ladite microalgue recombinante à au moins 150 %, de préférence au moins 200 % en comparaison avec une microalgue correspondante dans laquelle la voie de production de NO n'a pas été modulée.

2. Procédé selon la revendication 1, dans lequel ladite protéine NOA est la protéine NOA de *Phaeodactylum tricornutum* (*Pt*NOA) ayant une séquence d'acides aminés de SEQ ID N° : 2 ou un variant fonctionnel de celle-ci qui a une séquence d'acides aminés ayant une identité de séquence d'au moins 80 % avec SEQ ID N° : 2.

3. Procédé selon la revendication 1, dans lequel ladite protéine NOA est la protéine NOA de *Nannochloropsis gaditana* NOA (*Ng*NOA) ayant la séquence d'acides aminés de SEQ ID N° : 4 ou un variant fonctionnel de celle-ci qui a une séquence d'acides aminés ayant une identité de séquence d'au moins 80 % avec SEQ ID N° : 4.

4. Procédé selon l'une quelconque des revendications 1 à 3, dans lequel la microalgue est choisie parmi les Chromalveolata.

5. Procédé selon la revendication 4, dans lequel la microalgue est choisie parmi les Bacillariophyceae ou les Eustigmatophyceae.

6. Procédé selon la revendication 5, dans lequel la microalgue est Phaeodactylum *tricornutum.*

7. Procédé selon l'une quelconque des revendications 1 à 6, dans lequel la production de triacylglycérol survient pendant la phase de croissance de la microalgue recombinante.

8. Utilisation d'une microalgue recombinante qui a été transformée avec un acide nucléique recombinant codant pour une protéine NOA pour la production de TAG dans laquelle la teneur en triacylglycérol est accrue dans ladite microalgue recombinante à au moins 150 %, de préférence au moins 200 %, en comparaison avec une microalgue correspondante dans laquelle la voie de production de NO n'a pas été modulée.

9. Utilisation selon la revendication 8 pour la production d'acides gras, d'hydrocarbures ou d'alcools gras.

10. Utilisation selon l'une quelconque des revendications 8 ou 9 pour la production de biocarburant ou pour la production de cosmétiques.

11. Microalgue recombinante, qui a été transformée avec un acide nucléique recombinant codant pour un homologue de *Pt*NOA qui provient de *Nannochloropsis gaditana* et qui a la séquence d'acides aminés de SEQ ID N° : 4, dans laquelle la teneur en triacylglycérol est accrue dans ladite microalgue recombinante à au moins 150 %, de préférence au moins 200 %, en comparaison avec une microalgue correspondante dans laquelle la voie de production de NO n'a pas été modulée.
